(19) Europäisches Patentamt
European Patent Office
Office européen des brevets

(11) **EP 4 502 172 A1**

(12) **EUROPEAN PATENT APPLICATION**
published in accordance with Art. 153(4) EPC

(43) Date of publication:
**05.02.2025 Bulletin 2025/06**

(21) Application number: 23780982.7

(22) Date of filing: **30.03.2023**

(51) International Patent Classification (IPC):
*C12Q 1/06* $^{(2006.01)}$   *C12N 5/0783* $^{(2010.01)}$
*C12N 5/10* $^{(2006.01)}$   *G01N 15/14* $^{(2024.01)}$
*G01N 33/483* $^{(2006.01)}$   *G01N 33/543* $^{(2006.01)}$
*C12N 15/09* $^{(2006.01)}$

(52) Cooperative Patent Classification (CPC):
**C12N 5/06; C12N 5/10; C12Q 1/06; G01N 15/14;
G01N 33/483; G01N 33/543;** C12N 15/09

(86) International application number:
**PCT/JP2023/013374**

(87) International publication number:
**WO 2023/190974 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022059600
30.09.2022 JP 2022158218**

(71) Applicant: **Takeda Pharmaceutical Company
Limited
Osaka-shi, Osaka 541-0045 (JP)**

(72) Inventors:
• **OGAKI, Soichiro
Fujisawa-shi, Kanagawa 251-0012 (JP)**
• **IIDA, Tomomine
Fujisawa-shi, Kanagawa 251-0012 (JP)**

(74) Representative: **Jones, Nicholas Andrew
Withers & Rogers LLP
2 London Bridge
London SE1 9RA (GB)**

Remarks:
The complete document including Reference
Table(s) and the Sequence Listing(s) can be
downloaded from the EPO website

(54) **METHOD FOR PREDICTING GENE TRANSFER RATE**

(57)   Disclosed is a method for predicting the gene transfer efficiency of animal cells, the method comprising: (1) measuring the cell complexity of transgenic animal cells; and (2) predicting the gene transfer efficiency based on values measured in step (1).

Fig. 4

## Description

Technical Field

The present invention relates to a method for predicting gene transfer efficiency.

Background of Invention

In recent years, research and development of gene recombinant cellular medicines (transgenic/transfected cell preparations), such as chimeric antigen receptor (CAR)-T cells, has been actively conducted. For gene recombinant cellular medicines, such as CAR-T cells, the amount of the product is defined by multiplying the overall cell count by the percentage of transgene. Therefore, for CAR-T cell products, the CAR transfer efficiency is the most important index in the production process, shipping, and dosing. Conventionally, flow cytometry has been used to measure cell gene transfer efficiency; however, there is a problem in that it takes a long time (6 to 7 hours) from measurement to analysis, and requires complicated steps (a manual cell staining step). For example, NPL 1 has reported that CAR-T cells were measured by flow cytometry.

Citation List

Non-patent Literature

NPL 1: Cytometry Part B: Clinical Cytometry, Volume 100, Issue 2, pp. 218-224

Summary of Invention

Technical Problem

An object of the present invention is to provide, for example, a method for predicting gene transfer efficiency, the method being capable of measuring the gene transfer efficiency of cells more simply and efficiently than conventional methods.

Solution to Problem

As a result of extensive studies to achieve the above object, the present inventors found that by measuring intracellular complexity (e.g., SSC measured by flow cytometry) and using it as an index, the gene transfer efficiency of animal cells can be predicted easily and efficiently.

The present invention has been completed upon further research based on this finding, and provides, for example, the following method for predicting the gene transfer efficiency of animal cells.

[1] A method for predicting the gene transfer efficiency of animal cells, comprising:

(1) measuring the cell complexity of transgenic animal cells; and
(2) predicting the gene transfer efficiency based on values measured in step (1).

[2] The method according to [1], wherein the cell complexity is intracellular complexity.
[3] The method according to [1] or [2], wherein in step (2), the distribution of cell complexities is used to predict the gene transfer efficiency of a cell population.
[4] The method according to any one of [1] to [3], wherein in step (2), a parameter of the distribution of cell complexities is used to predict the gene transfer efficiency of the cell population.
[5] The method according to [4], wherein the parameter of the distribution of cell complexities is at least one member selected from the group consisting of mean, median, mode, variance, kurtosis, skewness, maximum value, minimum value, quartile, peak height, and half width.
[6] The method according to [4], wherein the parameter of the distribution of cell complexities is at least one member selected from the group consisting of mean, median, mode, variance, kurtosis, and skewness.
[7] The method according to [3], wherein in step (2), the distribution of complexities of transfected animal cells is determined from the distribution of cell complexities to thereby predict the gene transfer efficiency of the cell population.
[8] The method according to any one of [1] to [7], wherein the measurement of the cell complexity in step (1) is performed by measuring side scatter (SSC) or side fluorescence light (SFL) by a flow cytometer.
[9] The method according to any one of [1] to [8], wherein the measurement of the cell complexity in step (1) is performed by measuring SSC or SFL by a flow cytometer, and in step (2), SSC or SFL pulse height, width, and area are used to predict the gene transfer efficiency of the cell population.
[10] The method according to [1], [2], [8], or [9], wherein in step (2), cell size values are further used to predict the gene transfer efficiency of the cell population.
[11] The method according to [10], wherein forward scatter (FSC) values measured by a flow cytometer are used as the cell size values.
[12] The method according to [11], wherein in step (2), FSC pulse height, width, and area are used to predict the gene transfer efficiency of the cell population.
[13] The method according to any one of [1] to [7], wherein the cell complexity is cell surface complexity.

[14] The method according to [13], wherein the cell surface complexity is a circumference, solidity, unevenness, arithmetic average roughness, maximum height, ten-point average roughness, average spacing of unevenness, average spacing of local peaks, load length ratio, fractal dimension, aspect ratio, circularity, roundness, or compactness of the cells.

[15] The method according to any one of [1] to [7], wherein the measurement of the cell complexity in step (1) is performed by measuring intracellular organelles, cell surface glycans, cell membranes, cellular lipids, intracellular metabolites, cellular lipid droplets, or cell surface shapes.

[16] The method according to any one of [1] to [7], wherein the measurement of the cell complexity in step (1) is performed by measuring cell-derived autofluorescence.

[17] The method according to any one of [1] to [16], wherein the animal cells are immune cells.

[18] The method according to [17], wherein the immune cells are T cells or NK cells.

[19] The method according to any one of [1] to [16], wherein the animal cells are epithelial cells.

[19a] The method according to [19], wherein the animal cells are liver cancer cells.

[19b] The method according to any one of [1] to [19a], wherein the animal cells are human cells.

[20] The method according to any one of [1] to [19b], wherein a nucleic acid to be introduced is a nucleic acid encoding a chimeric antigen receptor.

[21] The method according to any one of [1] to [12] and [17] to [20], wherein the cell complexity difference between transfected cells and non-transfected cells is 5% or more for SSC or SFL values measured by a flow cytometer.

[22] A method for producing a transgenic cell preparation, comprising:

> (I) measuring the complexity of transgenic animal cells and measuring gene transfer efficiency based on measured values.

Advantageous Effects of Invention

According to the method for predicting gene transfer efficiency of the present invention, the gene transfer efficiency of cells can be measured with a simpler operation (e.g., the only operation required is automatic measurement using a measuring device) without the need for complicated steps, compared to conventional methods. Further, according to the method for predicting gene transfer efficiency of the present invention, the gene transfer efficiency of cells can be measured efficiently (e.g., in a short period of time of less than 5 minutes).

Brief Description of Drawings

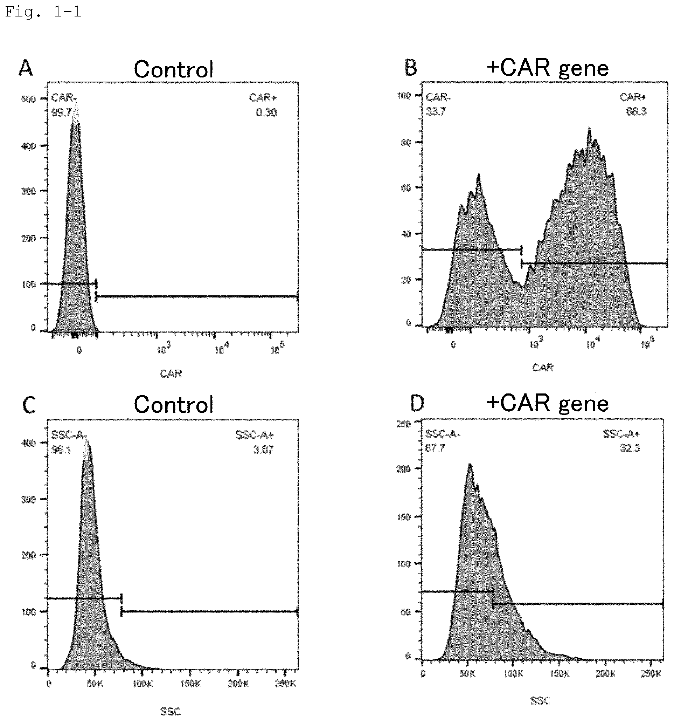

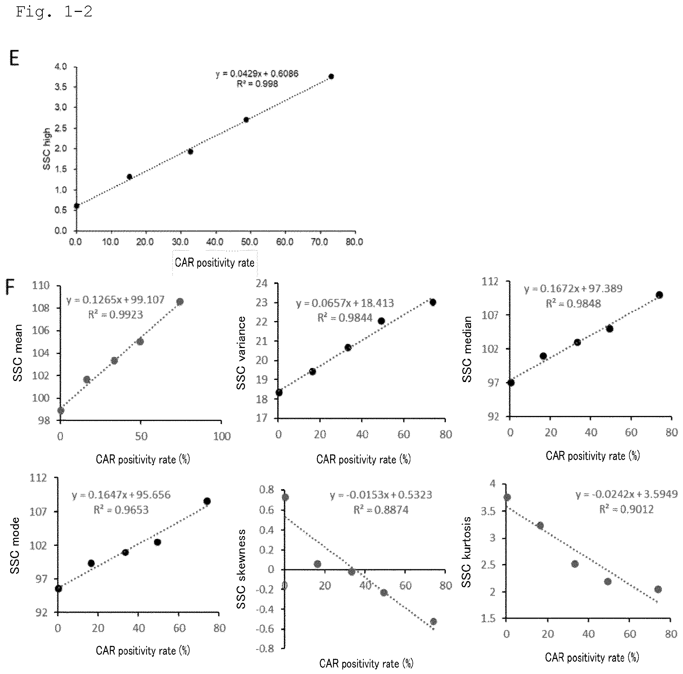

Fig. 1 is graphs showing the results of Example 1. (A) is a graph showing the CAR expression rate in untransduced T cells as a control. (B) is a graph showing the CAR expression rate in CAR-T cells transduced with CAR gene. (C) is a graph showing SSC in untransduced T cells as a control. (D) is a graph showing SSC in CAR-T cells transduced with CAR gene. (E) is a graph showing the correlation between the CAR positivity rate (%) and SSC high (the region obtained by gating on the area with visually high SSC from the histogram of the control) (N=5). (F) is graphs showing the correlation between the CAR positivity rate (%) and SSC mean, variance, median, mode, skewness, or kurtosis (N=5).

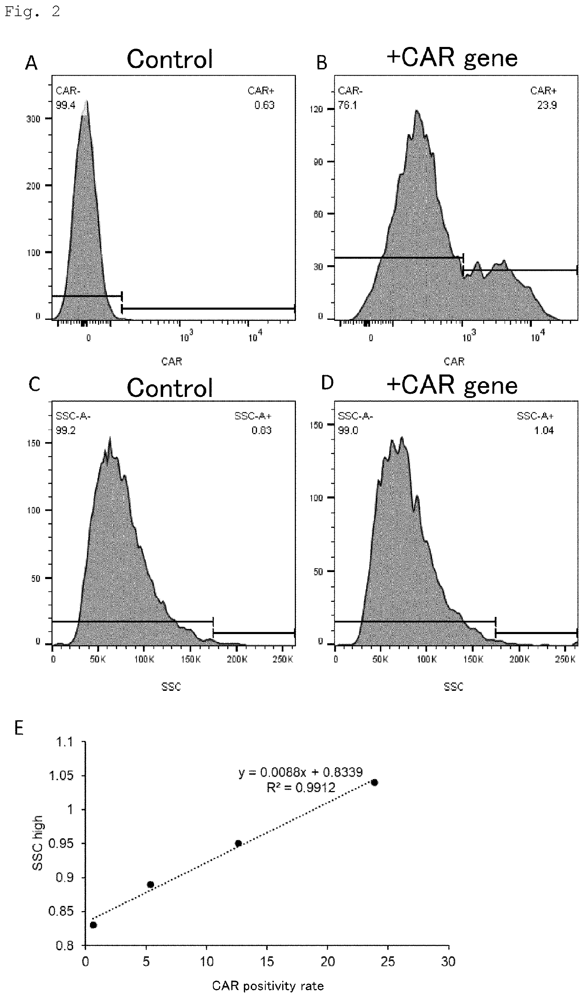

Fig. 2 is graphs showing the results of Example 2. (A) is a graph showing the CAR expression rate in untransduced SK-Hep-1 as a control. (B) is a graph showing the CAR expression rate in SK-Hep-1 transduced with CAR gene. (C) is a graph showing SSC in untransduced SK-Hep-1 as a control. (D) is a graph showing SSC in SK-Hep-1 transduced with CAR gene. (E) is a graph showing the correlation between the CAR positivity rate (%) and SSC high (N=4).

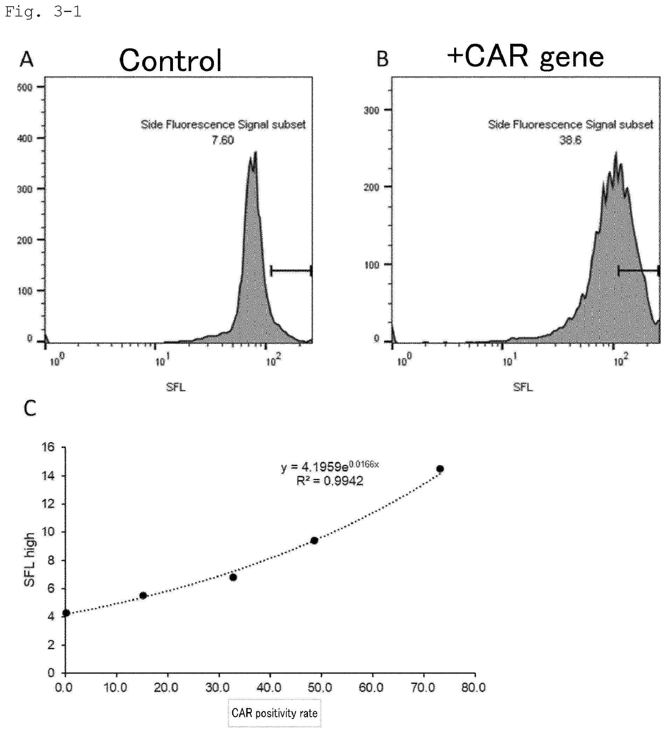

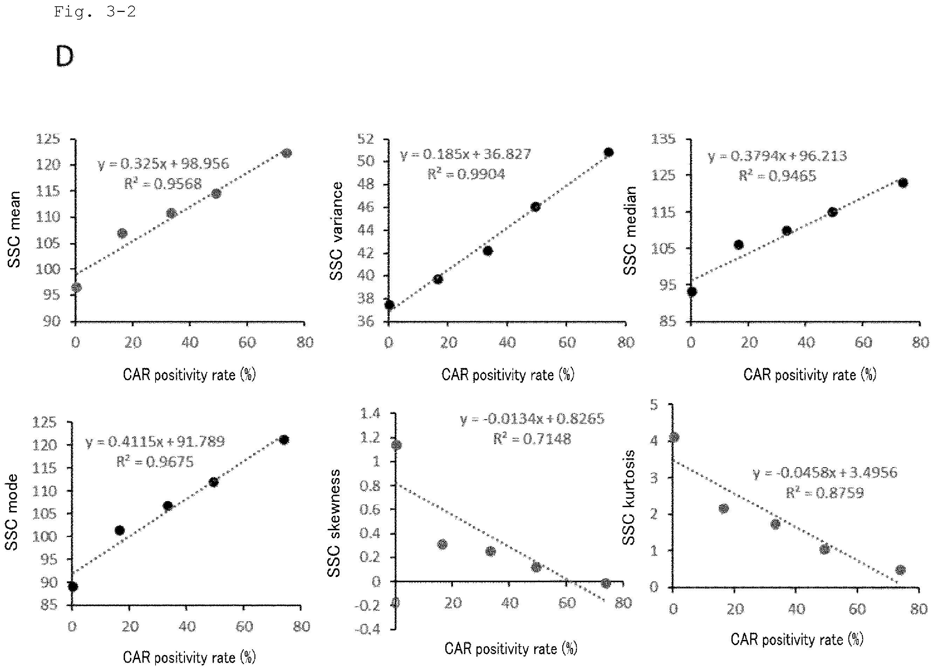

Fig. 3 is graphs showing the results of Example 3. (A) is a graph showing SFL in untransduced T cells as a control. (B) is a graph showing SFL in CAR-T cells transduced with CAR gene.

(C) is a graph showing the correlation between the CAR positivity rate (%) and SFL high (N=5). (D) is graphs showing the correlation between the CAR positivity rate (%) and SFL mean, variance, median, mode, skewness, or kurtosis (N=5).

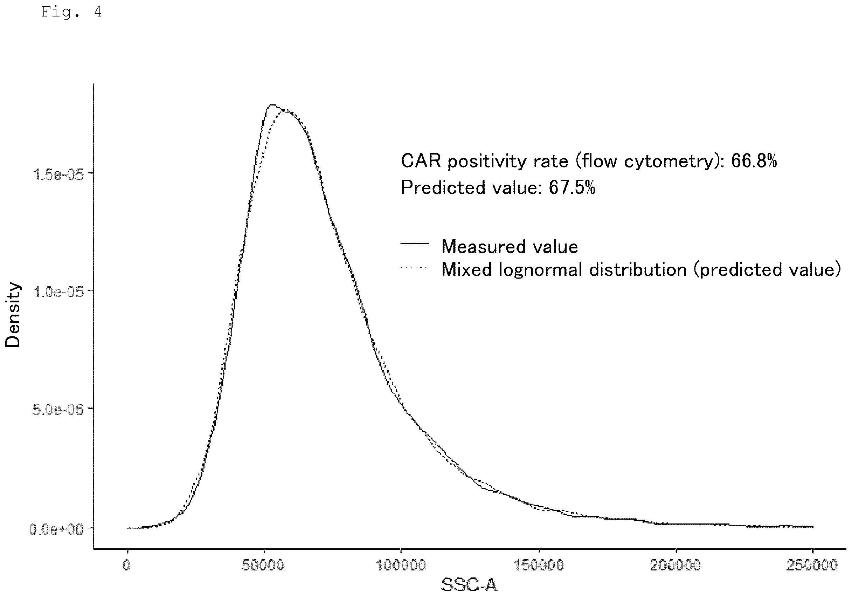

Fig. 4 is a graph showing the results of Example 4. The graph shows the distribution of SSC of cells transduced with CAR gene as determined from a mixed lognormal distribution model by using the SSC of cells transduced with CAR gene measured by flow cytometry. The CAR positivity rate predicted by flow cytometry was 66.8%, while the CAR positivity rate determined from the mixed lognormal distribution model was 67.5%.

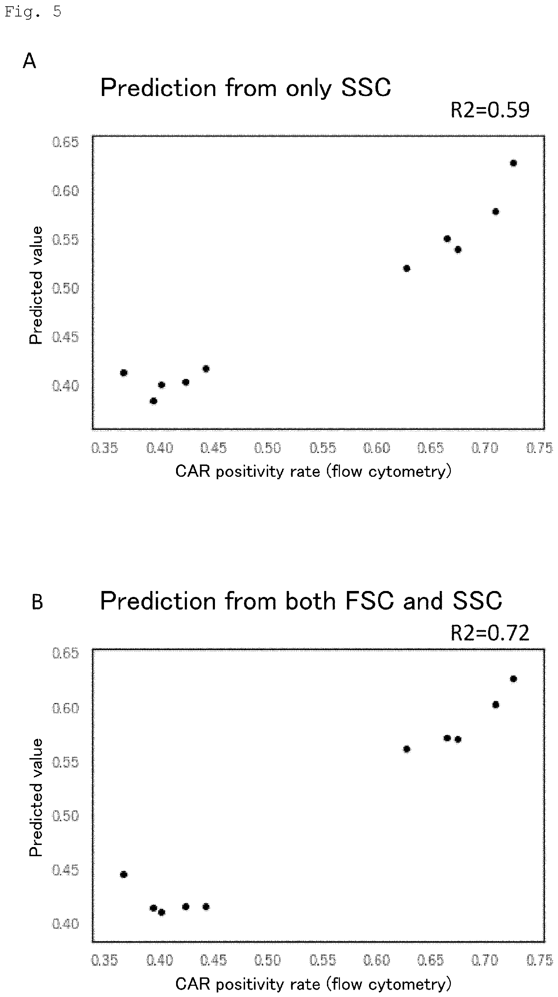

Fig. 5 is graphs showing the results of Example 5. (A) is a graph showing the CAR gene transfer efficiency (%) predicted by a machine learning model using SSC-H, SSC-W, and SSC-A, and the CAR gene transfer efficiency (%) measured by flow cytometry (N=18). (B) is a graph showing the CAR gene transfer efficiency (%) predicted by a machine learning model using SSC-H, SSC-W, SSC-A, FSC-H, FSC-W, and FSC-A, and the CAR gene transfer efficiency (%) measured by flow cytometry (N=18).

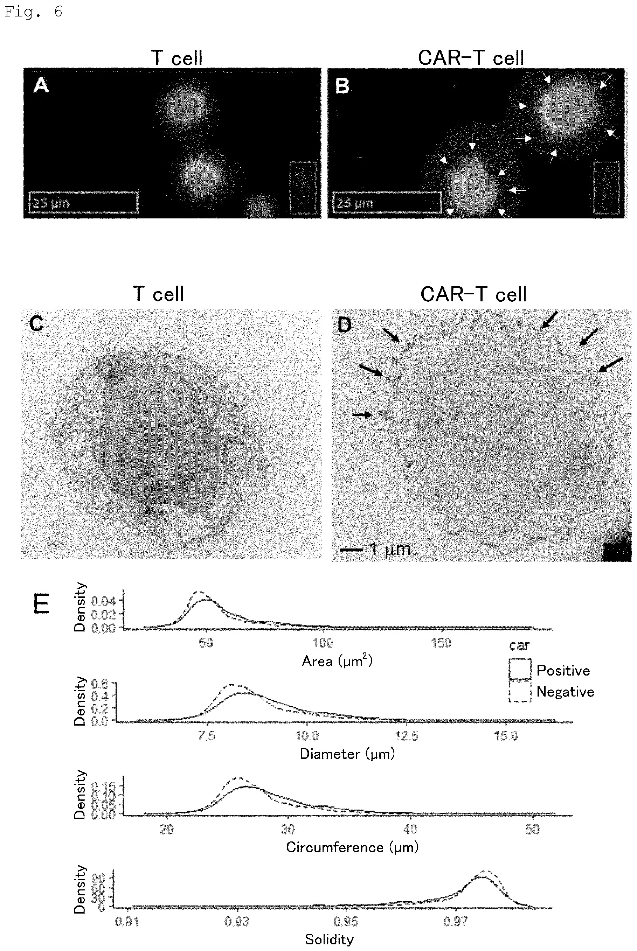

Fig. 6 shows the results of Example 6. (A) is an immunostaining image of untransduced T cells as a control. (B) is an immunostaining image of CAR-T cells transduced with CAR gene. (C) is an electron microscope image of untransduced T cells as a control. (D) is an electron microscope image of CAR-T cells transduced with CAR gene. (E) is graphs showing the area ($\mu$m$^2$), diameter ($\mu$m), circumference ($\mu$m), and solidity ($\mu$m) of untransduced T cells as a control and CAR-T cells transduced with CAR gene.

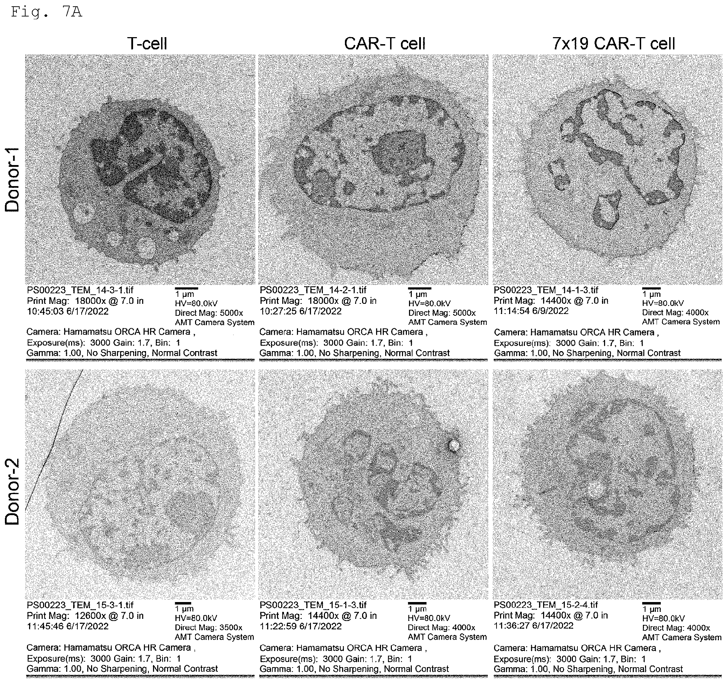

Fig. 7A is images showing the morphology of T cells and CAR-T cells in Example 7. (A): Transmission electron microscope images.

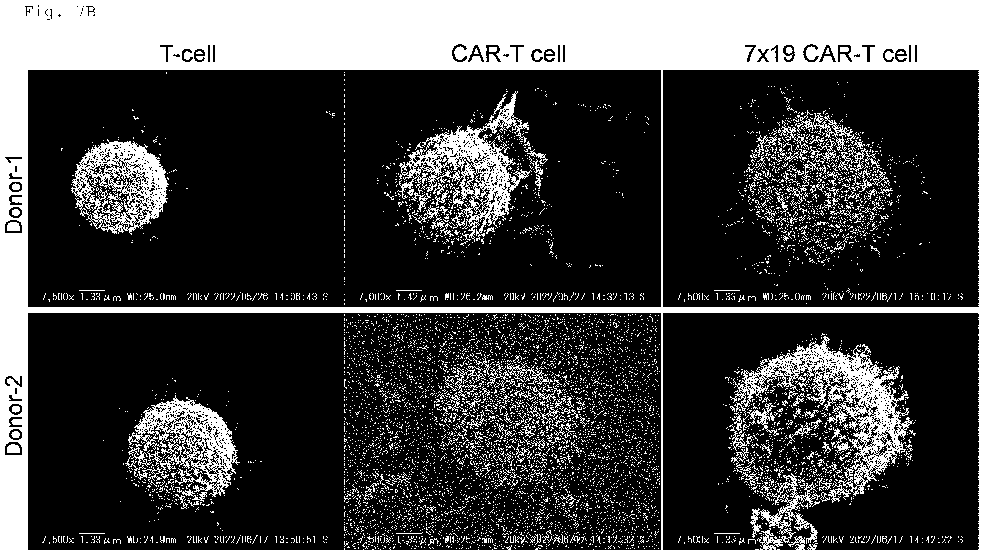

Fig. 7B is images showing the morphology of T cells and CAR-T cells in Example 7. (B): Scanning electron microscope images.

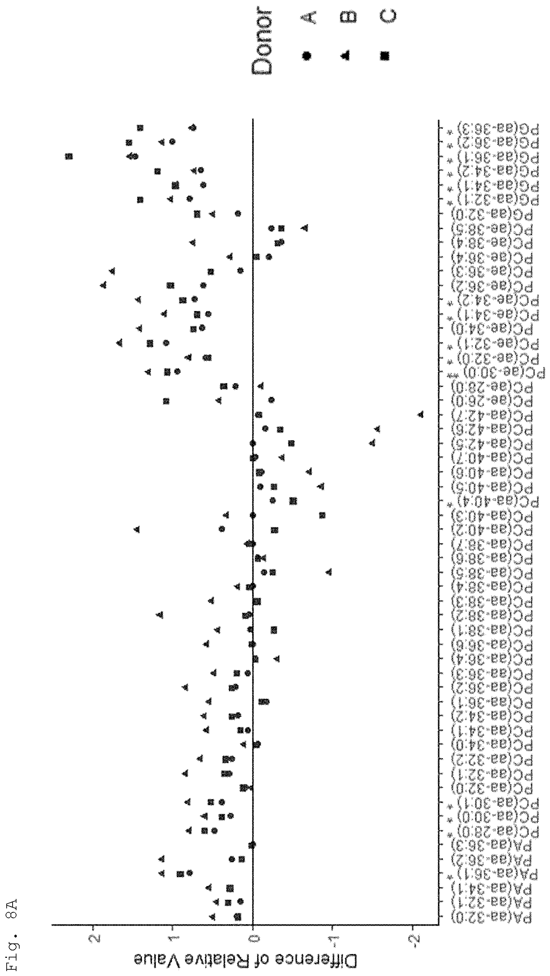

Fig. 8A is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

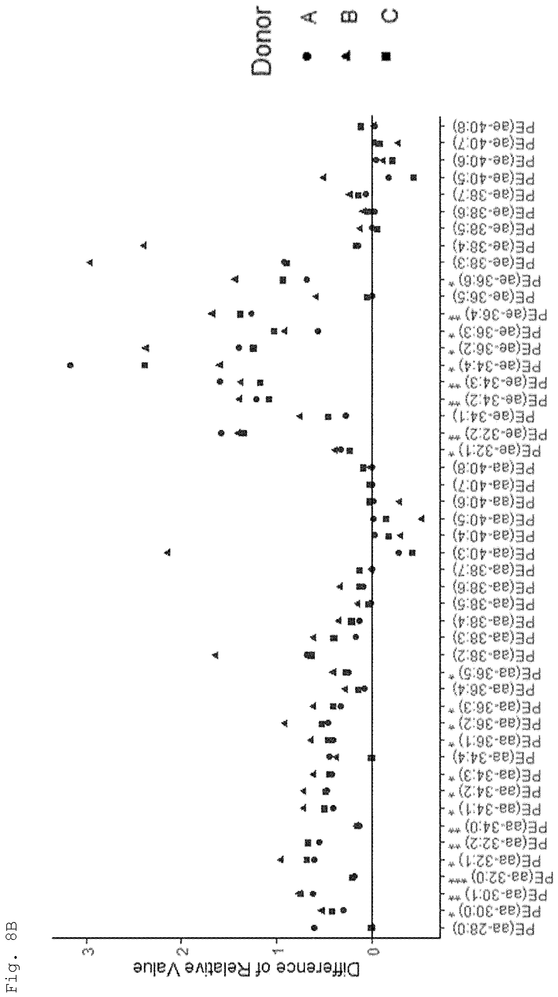

Fig. 8B is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

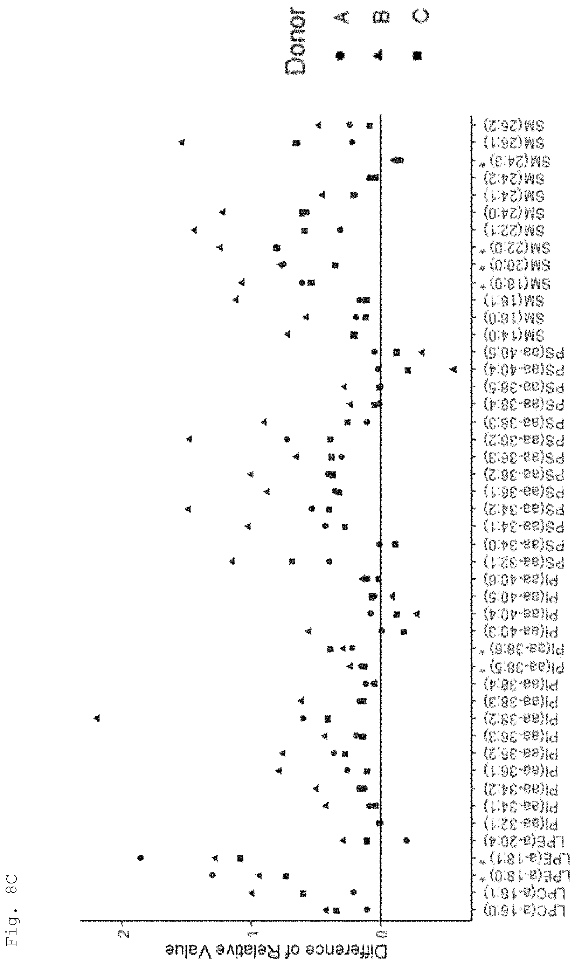

Fig. 8C is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

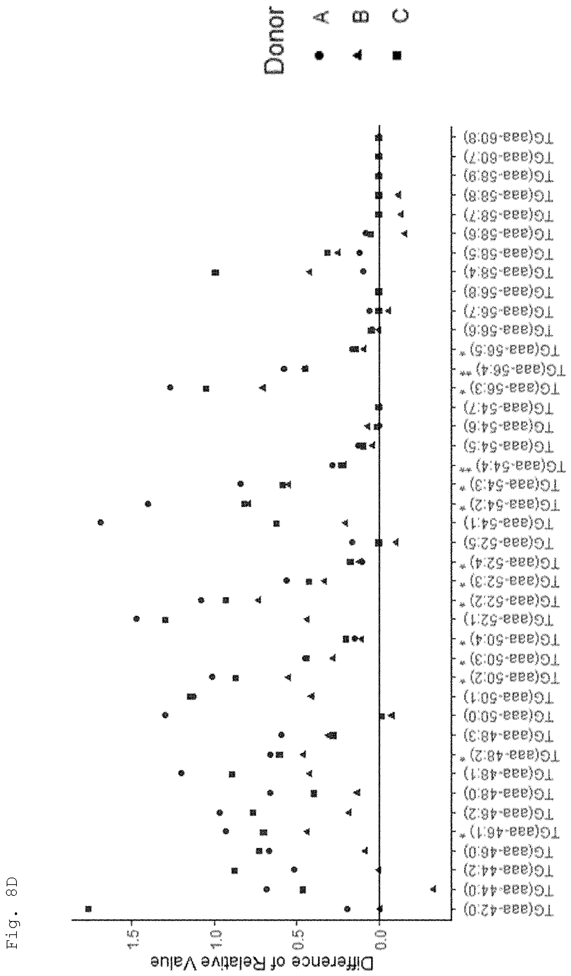

Fig. 8D is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

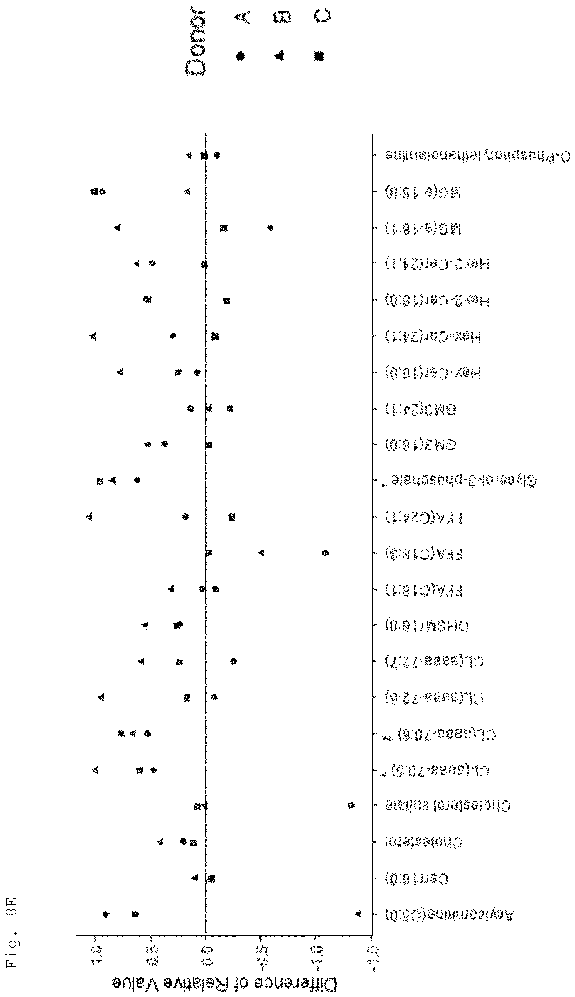

Fig. 8E is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

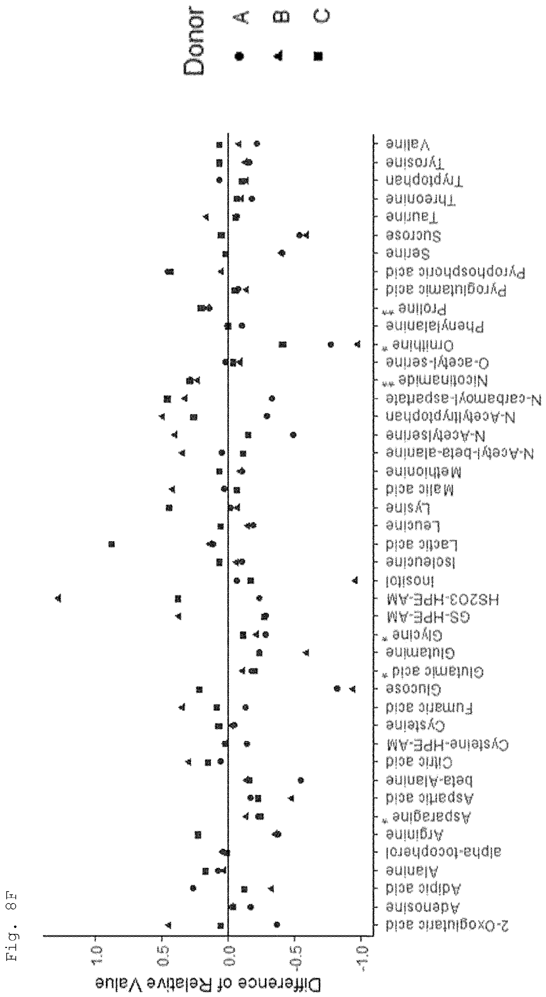

Fig. 8F is a graph showing the results of metabolomic and lipidomic analysis of T cells and CAR-T cells in Example 8. The horizontal axis represents each component, and the vertical axis represents the difference in amounts between T cells and CAR-T cells. A t-test was performed on each component. *: p<0.05, **: p<0.01, ***: p<0.001

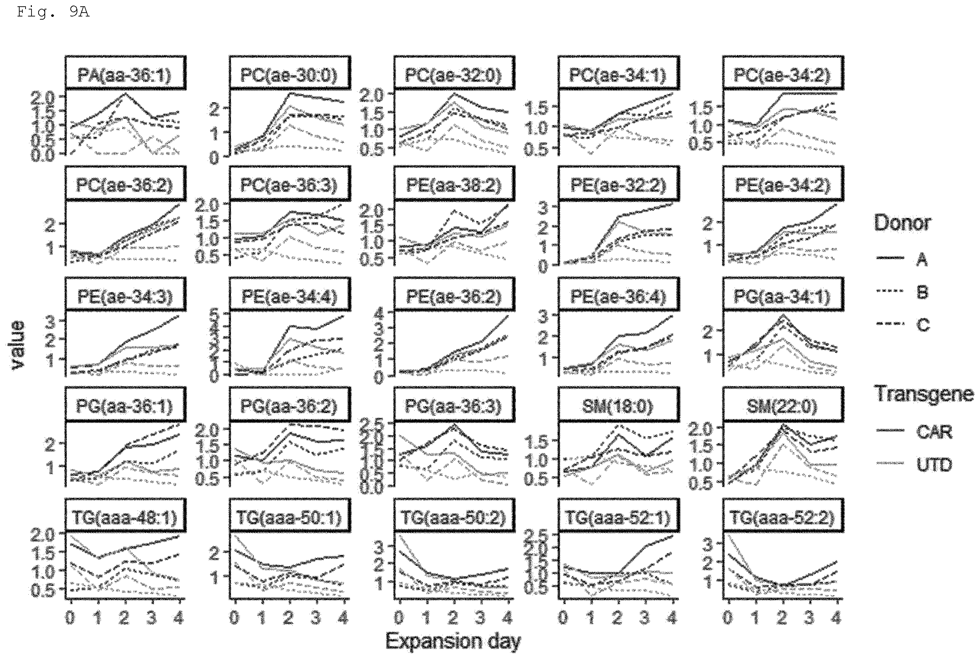

Fig. 9A is graphs showing the results of over-time metabolomic and lipidomic analysis of T cells and CAR-T cells in Example 8. For each component, a two-way analysis of variance was performed based on the number of days of culture and the type of cells, and changes in the amount of each component over time were plotted for the top 50 components with the lowest p-values.

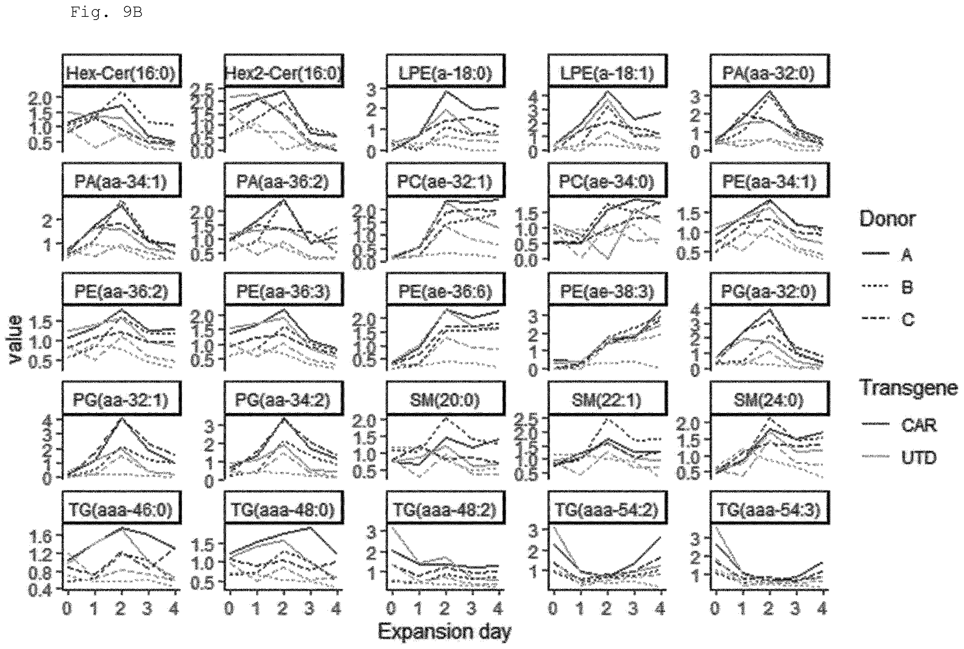

Fig. 9B is graphs showing the results of over-time metabolomic and lipidomic analysis of T cells and CAR-T cells in Example 8. For each component, a two-way analysis of variance was performed based on the number of days of culture and the type of cells, and changes in the amount of each component over time were plotted for the top 50 components with the lowest p-values.

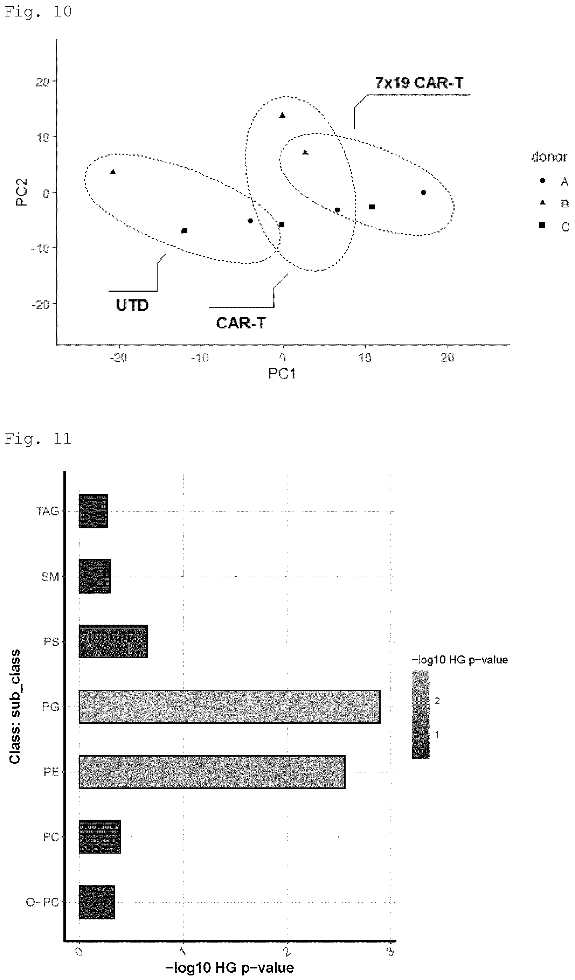

Fig. 10 is a graph showing the results of principal component analysis in terms of the metabolomic and lipidomic analysis of T cells and CAR-T cells in Example 8. Using each component, principal component analysis was performed, and a scatter plot was plotted with the first principal component on the horizontal axis and the second principal component on the vertical axis. The samples from each donor of UTD (T cells), CAR-T, and 7x19 CAR-T, are plotted in close positions and can be distinguished based on the presence or absence of CAR gene and their types.

Fig. 11 is a graph showing the results of enrichment analysis of metabolome and lipidome in Example 9.

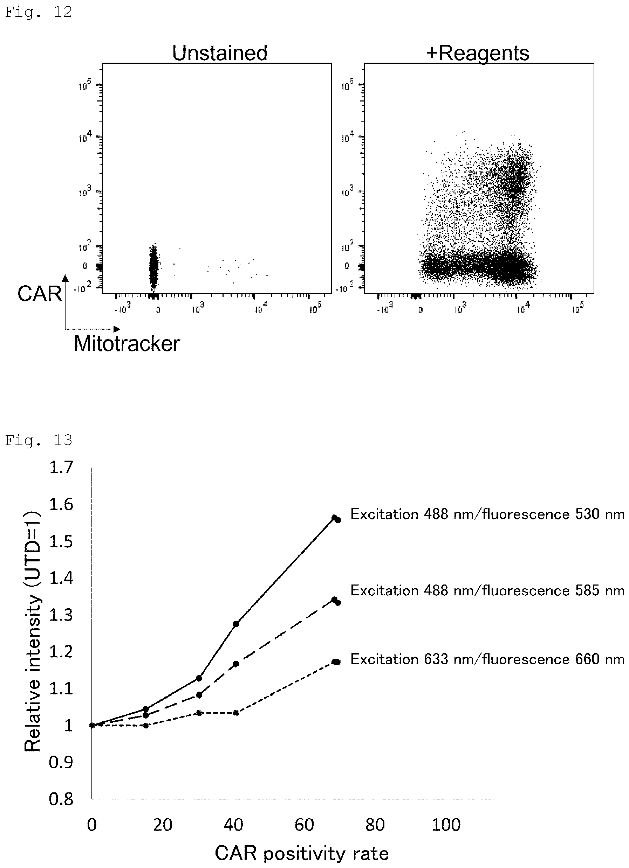

Fig. 12 is graphs showing the results of quantitative analysis of mitochondria in T cells and CAR-T cells in Example 9.

Fig. 13 is a graph showing the difference in autofluorescence between T cells and CAR-T cells in Example 9.

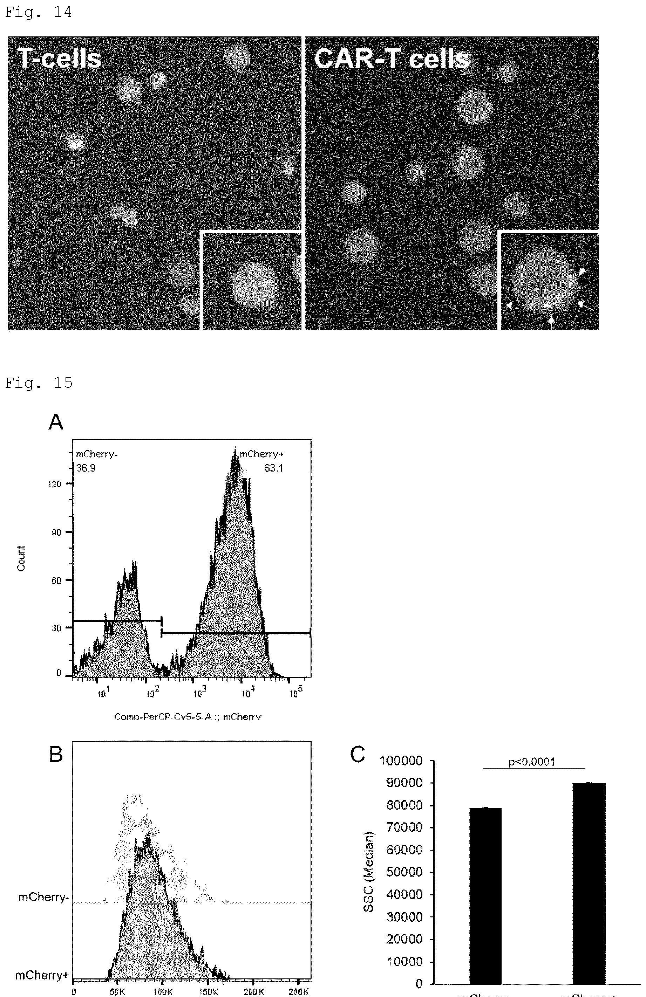

Fig. 14 is holographic microscope images of Example 10.

Fig. 15 is graphs showing the results of Example 11. (A) is a graph showing the fractionation by flow cytometry of $\gamma\delta$ T cells transduced with mCherry gene, (B) is a graph showing the SSC histograms of mCherry-negative cells and mCherry-positive cells, and (C) is a graph showing the median SSC of mCherry-negative cells and mCherry-positive cells (a t-test was performed on the median SSC) (N=4, error ranges are standard deviations).

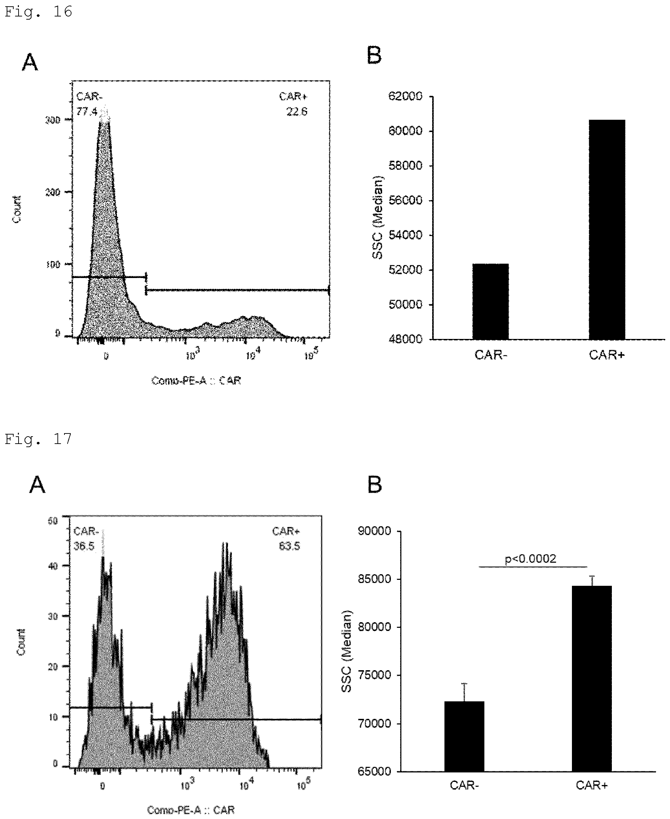

Fig. 16 is graphs showing the results of Example 12. (A) is a graph showing the fractionation by flow cytometry of $\gamma\delta$ T cells transduced with CAR gene, and (B) is a graph showing the median SSC of CAR-negative cells and CAR-positive cells.

Fig. 17 is graphs showing the results of Example 13. (A) is a graph showing the fractionation by flow cytometry of NK92 cells transduced with CAR gene, and (B) is a graph showing the median SSC of CAR-negative cells and CAR-positive cells (a t-test was performed on the median SSC) (N=4, error ranges are standard deviations).

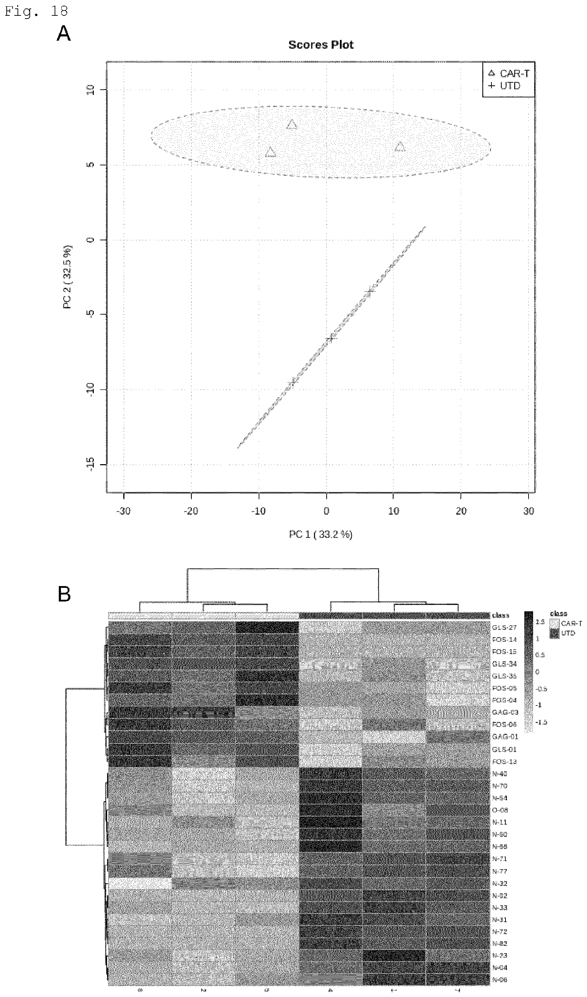

Fig. 18 is graphs showing the results of total glycomics analysis of T cells and CAR-T cells in Example 14. (A) shows the results of principal component analysis. (B) shows the results of heat map analysis. In the heat map analysis, the class indicates the fractionations of CAR-T cells and T cells (UTD), and each column indicates the expression levels of glycans in each cell by color.

Description of Embodiments

Embodiments of the present invention are described in detail below.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

In the present specification, "pluripotent stem cells" refer to embryonic stem cells (ES cells) and cells with similar pluripotency, i.e., cells with the potential to differentiate into various tissues (endoderm, mesoderm, and ectoderm) of a living body. Examples of cells with pluripotency similar to that of ES cells include induced pluripotent stem cells (also referred to as "iPS cells" in the present specification). If the pluripotent stem cells are ES cells or any cells derived from a human embryo, those cells may be produced by destroying the embryo or without destroying the embryo, and are preferably produced without destroying the embryo.

In the present specification, the term "cell population" refers to two or more cells of the same or different kind. The term "cell population" also refers to a mass of cells of the same or different kind.

The method for predicting the gene transfer efficiency of animal cells according to the present invention characteristically comprises:

(1) measuring the cell complexity of transgenic animal cells; and
(2) predicting the gene transfer efficiency based on values measured in step (1).

The term "gene transfer efficiency" in the present invention refers to the efficiency of transfer of nucleic acids into cells, and is, for example, the percentage of cells into which nucleic acids are transferred among all cells, the nucleic acid intake amount of the cell population, or the transgene expression rate of the whole cell population. When the transgene is a CAR, it is also referred to as the "CAR-positive rate."

- Step (1)

In step (1), the cell complexity of transgenic animal cells is measured.

The type of animal cells is not particularly limited, and various animal cells can be widely used. Examples of the type of animal cells include splenocytes, neurons, glial cells, pancreatic β cells, bone marrow cells, mesangial cells, Langerhans cells, epidermal cells, epithelial cells, endothelial cells, fibroblasts, fibrocytes, muscle cells (e.g., skeletal muscle cells, cardiac myocytes, myoblasts, and satellite cells), adipocytes, immune cells (e.g., macrophages, T cells, B cells, natural killer cells (NK cells), mast cells, neutrophils, basophils, eosinophils, monocytes, and megakaryocytes), synovial cells, chondrocytes, bone cells, osteoblasts, osteoclasts, mammary cells, hepatocytes, stromal cells, egg cells, and sperm cells, as well as stem cells that can be induced to differentiate into these cells (including neural stem cells, hematopoietic stem cells, mesenchymal stem cells, dental pulp stem cells, iPS cells, ES cells, and other pluripotent stem cells), progenitor cells, blood cells, oocytes, and fertilized eggs. T cells include αβ T cells, γδ T cells, helper T cells, cytotoxic T cells, regulatory T cells, suppressor T cells, tumor-infiltrating T cells, memory T cells, naive T cells, NK T cells, TCR-T cells, STAR receptor T cells, CAR-T cells, and the like. Further, animal cells also include primary cells, the above cells produced by inducing the in vitro differentiation of the above stem cells (e.g., iPS cells), and the like. In addition, animal cells also include various cancer cells. Animal cells may be contained singly or in a combination of two or more.

Organisms of origin of animal cells are not particularly limited, and examples of such organisms include mammals, such as humans, mice, rats, cows, horses, pigs, rabbits, dogs, cats, goats, monkeys, and chimpanzees. Preferred among these are humans.

Animal cells are animal cells into which an exogenous gene is introduced. The "exogenous gene" is a gene introduced from the outside into animal cells in order to express a desired protein, or monomer nucleotide, and can be suitably selected depending on the use of animal cells. The "transgenic animal cells" in the present invention include both animal cells that have actually been transfected with a gene and animal cells that have not been transfected with a gene as a result of an attempt of gene transfer. Further, in the present invention, the "transfected (animal) cells" refer to (animal) cells that have actually been transfected with a gene. "Transgenic cells" and "transfected cells" may be used interchangeably, depending on the context.

The exogenous gene can be, for example, a gene for expressing a chimeric antigen receptor (CAR).

The exogenous gene can be, for example, a gene for expressing a CAR and a gene for expressing a cytokine and/or a chemokine. As with general or known CARs, the CARs expressed by animal cells are basically configured such that peptides at sites of (i) an antigen recognition site that recognizes cell surface antigens of cancer cells (e.g., single-chain antibody, ligand, and peptide), (ii) a transmembrane region, and (iii) a signal transduction region that induces the activation of T cells, are linked via a spacer, as needed. Examples of other exogenous genes include T-cell receptors (TCRs), synthetic T-cell receptor and antigen receptor (STARs), chimeric T-cell antigen coupler (TAC) receptors, suicide genes (iCas9, HSV-TK, etc.), cytokines (interleukins, chemokines, etc.), and the like.

The means for introducing the exogenous gene into animal cells is not particularly limited, and various known or general means can be used. Typically, the exogenous gene is introduced into animal cells using an expression vector, and is expressed. The expression vector may be linear or cyclic, and may be a non-viral vector such as a plasmid, a viral vector, or a transposon vector.

The means for introducing the expression vector into animal cells can be made appropriate according to the embodiment. For example, the expression vector can be introduced into animal cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into animal cells by a virus infection method. Examples of viral vectors include retroviral vectors, lentiviral vectors, adenoviral vectors, and adeno-associated viral vectors. When using these viral vectors, a vector containing a desired exogenous gene and a packaging vector (plasmid) of each virus may be transfected into host cells using a corresponding commercially available kit to produce a recombinant virus, and then animal cells may be infected with the obtained recombinant virus.

When a plurality of exogenous genes are used, all of the exogenous genes may be contained in one expression vector, all of the exogenous genes may be separately contained in different expression vectors, or some of the plurality of exogenous genes may be contained in one expression vector, and the other genes may be separately contained in different expression vectors. When one expression vector contains a plurality of exogenous genes, the order in which those exogenous genes are arranged from the upstream side to the downstream side is not particularly limited.

The exogenous gene can be composed of a nucleic acid (polynucleotide) having a base sequence encoding the amino acid sequence of a desired protein or polypeptide. Those skilled in the art would be able to design and produce an expression vector capable of expressing a desired protein (polypeptide) in animal cells. The nucleic acids contained in the expression vector may be produced by chemically synthesizing DNA or may be produced (cloned) as cDNA.

In addition to the exogenous gene, the expression vector may contain sequences, such as promoter, terminator, enhancer, start codon, stop codon, polyadenylation signal, nuclear localization signal (NLS), and multi-cloning site (MCS), if necessary. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The "nucleic acid" may be any monomer nucleotide or any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

In another embodiment of the present invention, proteins, siRNAs, shRNAs, dsRNAs, miRNAs, antisense nucleic acids, and the like may be introduced into animal cells instead of the exogenous gene. Even when these substances other than the exogenous gene are introduced into animal cells, the gene transfer efficiency of the cells can be measured by measuring the cell complexity.

The "cell complexity" is not particularly limited and may be any complexity of cells. Examples include intracellular complexity, cell surface complexity, and the like. The cell complexity is preferably intracellular complexity.

The index used to indicate "intracellular complexity" is not particularly limited as long as it can represent intracellular complexity. Examples of the intracellular complexity include the complexity of intracellular structures, such as intracellular granular properties, the degree of nuclear lobulation, nucleus, intracellular organelles (e.g., mitochondria), membrane structures, chromosomes, chromatin, nucleosomes, oil droplets, and the like. Examples also include the complexity of cellular lipids, intracellular metabolites, intracellular glycans, and the like.

The index used to indicate "cell surface complexity" is not particularly limited as long as it can represent cell surface complexity. Examples of the index used to represent cell surface complexity include the circumference, solidity, unevenness, arithmetic average roughness, maximum height, ten-point average roughness, average spacing of unevenness, average spacing of local peaks, load length ratio, fractal dimension, aspect ratio, circularity, roundness, compactness, etc. of cells. The cell surface complexity may be, for example, the complexity of the cell surface shape, cell surface glycans, and the like.

The following are examples of the definition for each index mentioned above.

- Solidity = an area wrapped around the actual perimeter/an area wrapped around the envelope perimeter
- Unevenness: the degree of unevenness of the cell surface

- Arithmetic average roughness, maximum height, ten-point average roughness, average spacing of unevenness, average spacing of local peaks, and load length ratio: specified in JIS B 0601:1994 and JIS B 0031:1994.
- Fractal dimension: a statistical quantity that indicates how completely a fractal appears to fill space as it is enlarged to finer scales in fractal geometry.
- Aspect ratio = minor axis/maximum length
- Circularity = circular area perimeter/actual particle perimeter (or $4\pi$ (area)/(perimeter)$^2$)
- Roundness: the geometric tolerance of the roundness of a circle: JIS defines roundness as the amount of deviation of a circular object from a geometrically correct circle.
- Compactness = (perimeter)$^2$/area

The method for measuring the intracellular complexity is not particularly limited as long as it can measure the intracellular complexity, and examples include methods using various commercially available measuring devices, visual measurement methods using microscopes, image analysis using microscopes, and the like. Examples of such devices for measuring the intracellular complexity include flow cytometers, holographic microscopes, advanced DNA amount measuring devices, spectrophotometers, and electrophoresis systems. By measuring side scatter (SSC) with a flow cytometer, information about the intracellular complexity can be obtained from SSC; thus, SSC can be used as an index of intracellular complexity. Furthermore, by measuring side fluorescence light (SFL) with a flow cytometer, information about the amount of intracellular nucleic acids etc. stained with a fluorescent substance can be obtained from SFL; thus, SFL can be used as an index of intracellular complexity. The measurement of SFL is performed after staining cells with a fluorescent substance that stains nucleic acids (e.g., polymethine dyes, DAPI, PI, actinomycin dyes, such as 7-AAD, and Hoechst dyes, such as Hoechst 33342). The measurement of SFL may also be performed after staining cells with various known fluorescent substances that stain the cytoplasm, organelles, cell membrane, etc. The intracellular organelles can be specifically quantified with a holographic microscope. By quantifying the DNA amount with an advanced DNA amount measuring device, spectrophotometer, electrophoresis system, or the like, the DNA amount can be used as an index of intracellular complexity.

Examples of flow cytometers include products of Beckman Coulter, Inc. (e.g., Gallios, Navios, Navios EX, CytoFLEX, CytoFLEX S, CytoFLEX LX, Cytomics FC 500, and DxH500), BD Biosciences (e.g., BD FACSCalibur™ flow cytometer, BD EACSCanto™ II flow cytometer, BD EACSVerse™ flow cytometer, BD EACSLyric™ flow cytometer, BD LSRFortessa™ flow cytometer, BD LSRFortessa™X-20 flow cytometer, and BD FACSymphony™ flow cytometer), Thermo Fisher Scientific (e.g., Attune flow cytometer), Agilent Technologies (e.g., Novocyte flow cytometer), Sartorius (e.g., iQue3 flow cytometer), Sony Corporation (e.g., SA3800 and SP6800Z), Sysmex Corporation (e.g., Multiparameter Automated Hematology Analyzer XN-550, Multiparameter Automated Hematology Analyzer XN-450, Multiparameter Automated Hematology Analyzer XN-350, Multiparameter Automated Hematology Analyzer XN-330, Multiparameter Automated Hematology Analyzer pocH™-80i, and Multiparameter Automated hemacytometer XQ-320), and the like. Flow cytometry can measure intracellular complexity based on SSC and the cell size based on forward scatter (FSC). The measurement of the cell complexity and size using a flow cytometer can be performed according to a known method, for example, according to the manufacturer's manual of the flow cytometer.

Methods for measuring cell surface complexity are not particularly limited as long as they are capable of measuring cell surface complexity. Examples include measurement using various commercially available measuring devices, visual measurement using microscopes, image analysis of images acquired with a measuring device, and the like.

In addition, the cell complexity can be measured by measuring, for example, intracellular organelles, cell surface glycans, cell membranes, cellular lipids, intracellular metabolites, cellular lipid droplets, cell surface shapes, and the like. The cell complexity can also be measured by measuring cell-derived autofluorescence (e.g., by a flow cytometer, a microscope, or a microplate reader). Autofluorescence is the natural emission of light (photoluminescence) that occurs when biological structures such as mitochondria and lysosomes absorb light, and is used to distinguish the light originating from artificially added fluorescent markers (fluorophores).

The following are examples of the definition for each index mentioned above.

- Intracellular organelles refers to membrane-compartmentalized structures present in eukaryotic cells (e.g., mitochondria, endoplasmic reticulum, Golgi apparatus, lysosomes, vacuoles, endosomes, and peroxisomes). Gene transfer, for example, increases the amount of mitochondria.
- Cell surface glycans include N-linked glycans, O-linked glycans, glycolipid glycans, glycosaminoglycans, and free oligosaccharides. Although most glycans are present outside cells, they may basically be either intracellular or extracellular glycans. Gene transfer, for example, increases glycolipid glycans, glycosaminoglycans, and free oligosaccharides, and decreases N-linked glycans and O-linked glycans.
- Cell membrane refers to a biological membrane that separates cells from the outside environment. Gene transfer, for example, changes the components that make up a cell membrane and increases the amount of cell membrane.
- Cellular lipids refer to the lipid components of cells, especially lipids contained in a cell membrane. Gene transfer, for example, changes cellular lipid components and increases phosphatidic acid, phosphatidylcholine, phosphatidylethanolamine, phosphatidylglycerol, sphingomyelin, triacylglycerol, hexosylceramide, dihexosylceramide, lysophosphatidylethanolamine, and the like.
- Intracellular metabolites refer to metabolic intermediates present in cells. Gene transfer changes, for example, cellular metabolic components.
- Cellular lipid droplets are intracellular organelles that are present in cells and store lipids. Gene transfer, for example, increases the amount of lipid droplets.
- The cell surface shape can be expressed by, for example, the degree of unevenness. Gene transfer, for example, changes the cell surface shape to become more uneven and more complex (the circumference becomes longer and the solidity becomes smaller).

- Step (2)

In step (2), the gene transfer efficiency is predicted based on values measured in step (1).

Since the correlation between the cell complexity and the gene transfer efficiency was confirmed in the Examples described below, the measured cell complexity values can be used to predict the gene transfer efficiency. In the present invention, the term "prediction" is used in the same sense as "measurement," and these terms can be used interchangeably.

In step (2), the distribution of cell complexities can be used to predict the gene transfer efficiency of a cell population. In one embodiment, the ratio of high-cell complexity regions, compared to the distribution of complexities of control cells without gene transfer, is used to predict the gene transfer efficiency of the cell population. In another embodiment, parameters of the distribution of cell complexities are used to predict the gene transfer efficiency of the cell population. Such parameters of the distribution of cell complexities are not particularly limited, and examples include the mean, median, mode, variance (standard deviation), skewness, kurtosis, maximum value, minimum value, quartile, peak height, and half width of the distribution measured in step (1) (in particular, the mean, median, mode, variance (standard deviation), skewness, and kurtosis). The mean, median, mode, and variance (standard deviation) are positively correlated with the gene transfer efficiency, while the skewness and kurtosis are negatively correlated with the gene transfer efficiency.

As the method for using cell complexity distribution parameters to predict the gene transfer efficiency of the cell population, for example, a calibration curve is created based on the gene transfer efficiency of cells whose gene transfer efficiency is known and cell complexity distribution parameters, and the calibration curve is used to determine the gene transfer efficiency from parameters obtained from the values measured in step (1). The calibration curve can be created by an ordinary method, such as the least squares method, using software or the like.

As another embodiment of step (2), the distribution of complexities of transfected animal cells is determined from the cell complexity distribution to predict the gene transfer efficiency of the cell population. For example, the gene transfer efficiency of the cell population is predicted by using a mixed lognormal distribution model to determine the ratio of two populations, a transfected cell population and a non-transfected cell population, to the whole cell population. Specifically, each parameter of the mixed lognormal distribution model is estimated by fitting a mixed normal distribution to the log-transformed data of the values measured in step (1) using the maximum likelihood method, and the gene transfer efficiency of the cell population is determined (for details, see the Examples described below). Such a calculation process can be performed by using known software, such as R, SAS, SPSS, or JMP.

As still another embodiment of steps (1) and (2), the gene transfer efficiency of the cell population is predicted by performing the cell complexity measurement in step (1) by measuring SSC or SFL by a flow cytometer, thereby using SSC or SFL pulse height, width, and area in step (2) for the prediction of the gene transfer efficiency of the cell population. That is, SSC or SFL pulse height, width, and area measured in the individual cells are used to predict the gene transfer efficiency. Using multiple values in this way enables robust analysis. In addition to the cell complexity values, cell size values (e.g., FSC measured by a flow cytometer, in particular, FSC pulse height, width, and area) may also be used to predict the gene transfer efficiency. A combined use of cell complexity values and cell size values in this way allows for a more robust analysis. The gene transfer efficiency of the cell population can be predicted by, for example, using a machine learning model. That is, learning data with known gene transfer efficiency are given to a prediction model to learn the data to create a learned prediction model, and then animal cell complexity values are input into the learned prediction model to predict the gene transfer efficiency. As a specific example, a learned prediction model is created using the data determining the presence or absence of gene transfer in the individual cells as correct labels, and the measurement values of SSC or SFL pulse height, width, and area as learning data, and then the measured values of SSC or SFL pulse height, width, and area of the individual cells are input into the learned prediction model to predict the gene transfer efficiency. As the learning data, SSC or SFL distribution parameters of the cell population may be used in addition to measured values of SSC or SFL pulse height, width, and area of the individual cells. Since the prediction gives the probability of being classified as transfected cells, the presence or absence of gene transfer in the individual cells may be determined by a specific threshold (e.g., 50%), and the gene transfer efficiency of the cell population may be determined. Alternatively, the mean probability of being classified as transfected cells for the individual cells may be used as the gene transfer efficiency of the cell population. The machine learning model may be one capable of performing regression analysis, and examples include lasso regression, ridge regression, elastic-net regression, principal component regression, partial least squares regression, random forest, gradient boosting decision tree, neural network, deep learning, support vector machine, and the like.

As still another embodiment of step (2), the gene transfer efficiency of the cell population is predicted by using, in addition to the values measured in step (1), values measured at least at one stage before and after gene transfer to predict the gene transfer efficiency of the cell population. That is, the complexity of animal cells is measured in multiple steps for producing transgenic animal cells, and animal cell complexity values at multiple time points, not just one, are used to predict the gene transfer efficiency. Using cell complexities at multiple time points in this way enables robust analysis. When predicting the gene transfer efficiency using animal cell complexity values at multiple time points, the number of multiple time points is, for example, 2, 3, 4, 5, 6, 7, 8, 9, or 10. Specific examples of multiple time points include raw materials, after activation of the cells, after gene transfer into the cells, after expanded culture after gene transfer, and the like. The animal cell complexity values used herein are preferably the cell complexity distribution parameters described above. In addition to the cell complexity values, cell size values (e.g., FSC measured by a flow cytometer) may also be used to predict the gene transfer efficiency. A combined use of cell complexity values and cell size values in this way allows for a more robust analysis. The gene transfer efficiency of the cell population can be predicted by, for example, using a machine learning model. That is, learning data with known gene transfer efficiency are given to a prediction model to learn the data to create a learned prediction model, and then animal cell complexity values measured at multiple time points are input into the learned prediction model to predict the gene transfer efficiency. The machine learning model may be one capable of performing regression analysis, and examples include lasso regression, ridge regression, elastic-net regression, principal component regression, partial least squares regression, random forest, gradient boosting decision tree, neural network, deep learning, support vector machine, and the like.

The difference in complexity (particularly diameter, and SSC or SFL values measured by a flow cytometer) between transfected cells (animal cells actually transfected with a gene) and non-transfected cells (animal cells not transfected with a gene) is such that, particularly among the complexity distribution parameters of the transfected cell population and non-transfected cell population, one or more of mean, median, and mode (particularly median) are, for example, 5% or more, 6% or more, 7% or more, 8% or more, 9% or more, or 10% or more. The complexity of transfected cells is larger than that of non-transfected cells.

The method for producing a transgenic cell preparation of the present invention (also referred to simply as "the production method of the present invention" in the present specification) characteristically comprises:

(I) measuring the complexity of transgenic animal cells, and measuring the gene transfer efficiency based on the measured values.

The production method of the present invention may further comprise:
(II) introducing a gene into an animal cell.

Steps (I) and (II) of the production method of the present invention can be carried out by the method described above. The cell preparation produced by the production method of the present invention (hereinafter also referred to as "the cell preparation of the present invention") is preferably produced as a parenteral preparation, for example, by mixing an effective amount of transgenic animal cells with a pharmaceutically acceptable carrier according to known means (e.g., the methods described in the Japanese Pharmacopoeia). The cell preparation of the present invention is preferably produced as a parenteral preparation, such as injection, suspension, or infusion. Examples of parenteral administration methods include intravenous, intraarterial, intramuscular, intraperitoneal, or subcutaneous administration. Examples of the pharmaceutically acceptable carrier include solvents, bases, diluents, excipients, soothing agents, buffers, preservatives, stabilizers, suspensions, isotonic agents, surfactants, solubilizing agents, and the like.

The dose of the cell preparation of the present invention can be suitably determined depending on various conditions, such as patient's body weight, age, sex, and symptoms. The cell preparation of the present invention may be administered once or several times. The cell preparation of the present invention can have a known form suitable for parenteral administration, such as injection or infusion. Further, the cell preparation of the present invention may contain physiological saline, phosphate buffered saline (PBS), medium, and the like in order to stably maintain the cells. Examples of the medium include RPMI, AIM-V, X-VIVO10, and other media, but are not limited thereto. In addition, pharmaceutically acceptable carriers (e.g., human serum albumin), preservatives, and the like may be added to the cell preparation for the purpose of stabilization. The cell preparation of the present invention is applied to mammals, including humans.

In contrast to conventional methods for measuring the gene transfer efficiency by flow cytometry, which requires complicated manual work to stain the transgene with antibodies, the method for predicting gene transfer efficiency of the present invention does not require such complicated steps and makes it possible to measure the gene transfer efficiency of cells with a simple operation, such as automatic measurement using a measuring device, only by introducing samples into, for example, a flow cytometer, in particular, a hematology analyzer or other medical devices. Further, the method for predicting gene transfer efficiency of the present invention makes it possible to measure the gene transfer efficiency of cells efficiently, for example, in a short period of time of less than 5 minutes, whereas conventional methods take 6 to 7 hours. In addition, the method of the present invention and the cell type are not particularly limited, and the method can be applied to various cells. It is expected that the method of the present invention will be applied to gene therapies, such as CAR-T cell therapy.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

Examples

Examples are provided below in order to explain the present invention in more detail. However, the present invention is not limited to these Examples.

Method

Medium

CAR-T cell culture medium: 2.6% OpTmizer Expansion Basal Supplement (Thermo Fisher Scientific), 1% L-Glutamine (Thermo Fisher Scientific), 1% Streptomycin, and 2% CTS Immune Cell SR (Thermo Fisher Scientific) were added to OpTmizer CTS T-Cell Expansion basal medium (Thermo Fisher Scientific) to produce a basal medium. MACS GMP IL-2 (Miltenyi Biotec) was added thereto at 20 IU/mL or 40 IU/mL.

SK-HEP-1 cell culture medium: 10% FBS (Biosera), 1% Non-essential amino acids (FUJIFILM Wako Pure Chemical Corporation), 1% Penicillin-Streptomycin solution (FUJIFILM Wako Pure Chemical Corporation), and 1 mM Sodium pyruvate (FUJIFILM Wako Pure Chemical Corporation) were added to MEM, L-Gln (+) (Thermo Fisher Scientific) for production.

NK92 cell culture medium: 20% FBS (Biosera) was added to RPMI 1640 Media (Thermo Fisher Scientific), and MACS GMP IL-2 (Miltenyi Biotec) was added at 200 IU/mL for production.

Gene transfer into T cells

After thawing Leukopak (HemaCare) or $\gamma\delta$ T cells (HemaCare), the cells were diluted in the CAR-T cell culture medium to a concentration of $2.0\times10^6$ cells/mL or less (raw materials before production). The cells were seeded in a culture bag so that the ratio of cell suspension to MACS GMP T-Cell TransACT (Miltenyi Biotec) was 17.5:1, and cultured for more than 36 hours to less than 48 hours (cell activation step). The activated cells were diluted in the culture medium using the LOVO Cell Processing System (Fresenius Kabi) or a centrifuge, and seeded at $6.07\times10^5$ cells/cm² or less in a culture bag that was pre-coated with Retronectin (trademark) (Takara Bio Inc.) and retrovirus with CAR gene, with CAR gene, IL-7 gene, and CCL19 gene, or with mCherry gene, and cultured until the next day (gene transfer step). The cells were seeded at $2.2\times10^6$ cells/cm² or less in a culture bottle (G-REX, Wilson Wolf), and cultured for 3 to 7 days, thereby producing CAR-T cells (final product). The CAR gene used has a base sequence represented by SEQ ID NO: 1, the CAR gene, IL-7 gene, and CCL19 gene used have a base sequence represented by SEQ ID NO: 2, and the mCherry gene used has a base sequence represented by SEQ ID NO: 3 (the same applies to the following case of SK-HEP-1 cells).

CAR gene transfer into SK-HEP-1 cells

SK-HEP-1 cells (ATCC) were diluted in the SK-HEP-1 cell culture medium to a concentration of $2.0\times10^6$ cells/mL or less, seeded at $6.07\times10^5$ cells/cm² or less on a culture plate that was pre-coated with Retronectin (Takara Bio Inc.) and retrovirus with CAR gene, and cultured for 4 days, thereby introducing the CAR gene into the SK-HEP-1 cells.

Production of CAR-NK cells

After thawing NK-92 (ATCC), which is a cell line of NK cells, the cells were cultured in the NK92 cell culture medium. After culture, the cell suspension was seeded at $6.07\times10^5$ cells/cm² or less in a culture bag that was pre-coated with Retronectin (trademark) (Takara Bio Inc.) and retrovirus with CAR gene, thereby producing CAR-NK cells.

Measurement of intracellular complexity, cell size, and gene transfer efficiency using flow cytometry

Using a BD FACS Canto II flow cytometer (BD Biosciences), gene transfer efficiency was measured using CAR antigen or mCherry fluorescence. The intracellular complexity was quantified by SSC, and the cell size was quantified by FSC.

Measurement of intracellular complexity using hematology analyzer

Using a multiparameter hematology analyzer XN-330 (Sysmex Corporation), the intracellular complexity was quantified by SFL.

Cell distribution analysis using mixed lognormal distribution model

Using statistical analysis software R, each parameter of a mixed lognormal distribution model was estimated by fitting a mixed normal distribution to log-transformed data of SSC of individual cell measured by flow cytometry using the maximum likelihood method. The probability density function of the two-group mixed normal distribution is represented as follows:

$$p(x) = \lambda_1 N(x|\mu_1, \sigma_1) + \lambda_2 N(x|\mu_2, \sigma_2)$$

wherein x is the natural logarithm of each measured value of cell SSC,

$$N(x|\mu, \sigma)$$

represents the normal distribution of mean $\mu$ and variance $\sigma$, $\lambda$ is a weighting factor in the mixture distribution, and $\lambda_1+\lambda_2=1$. Each parameter was estimated by maximum likelihood using the EM algorithm so that the following log likelihood function of the probability density function p(x) was maximized:

$$\ln L(\lambda, \mu, \sigma|x)$$

Since the ratios of the two populations to the total cell population were $\lambda_1$ and $\lambda_2$, the weighting factor $\lambda$ of the larger population of $\mu_1$ and $\mu_2$ was estimated as the ratio of target transfected cells.

Prediction of gene transfer efficiency using machine learning model

Using the programming language Python in Jupyter Notebook, which is an integrated development environment that runs on a web browser, a machine learning model was constructed by the random forest algorithm to predict the presence or absence of CAR gene transfer from the SSC values of individual cells. One sample was measured by flow cytometry using CAR antigen, and a prediction model was constructed using the data determining whether individual cells were CAR positive or negative from the CAR antigen as correct labels, and the measured SSC height, width, and area values (SSC-H, SSC-W, SSC-A) as training data. The prediction gives the probability of individual cells being classified as CAR positive. The mean probability of being classified as CAR positive for all the measured cells was used as the predicted value of the CAR positivity rate in the measurement, and the measured SSC values of 16 samples other than those used for prediction were used as testing data and fitted to the model to predict the CAR positivity rate of each sample.

Similarly, a prediction model was constructed from one sample using all the measured FSC and SSC values, which are parameters obtained by flow cytometry without staining, and the CAR positivity rate of the 16 samples other than those used for prediction was predicted.

Structural analysis of cell membrane using antibody staining

After fixing, the cells were stained with anti-CD3 antibody (Abcam) and anti-CAR antibody, and then analyzed by Nanozoomer (Hamamatsu Photonics K.K.). The area, diameter, circumference, and solidity, which indicates the smoothness of particles, of each cell were calculated from the captured images using the image analysis software Image J.

Structural analysis of cell membrane with immunoelectron microscopy

After fixing, the cells were stained with an anti-CAR antibody, and then double-stained with uranyl acetate and a lead citrate staining solution, followed by analysis with an electron microscope (H-7600, Hitachi High-Tech Corporation).

Separation of CAR-T cells by MACS

The cells were labeled with CAR antibody and Anti-PE MicroBeads UltraPure (Miltenyi Biotec) and then separated using a MACS MultiStand (Miltenyi Biotec) and QuadroMACS™ Separator (Miltenyi Biotec).

Imaging by transmission electron microscope

The cells were solidified into a gel-like state by using iPGell (GenoStaff Co., Ltd.). After fixation with a 2.5% glutaraldehyde phosphate buffer, post-fixation was performed with 1% osmic acid. Subsequently, a resin-embedded block was produced in a standard manner and cut into ultrathin sections at 80 $\mu$m. The thinly cut sections were heavily stained with uranyl acetate and a lead citrate staining solution, and observed under an electron microscope (H-7600, Hitachi High-Tech Corporation).

Imaging by scanning electron microscope

Unfrozen cells were seeded onto Poly-L-Lysine-coated cover glasses, allowed to stand, and fixed with 1% glutaraldehyde phosphate buffer. After post-fixation with 1% osmium acid, dehydration, drying, and gold deposition were performed in a standard manner. The prepared specimens were observed under a scanning electron microscope (VE8800, Keyence Corporation).

Metabolomic and lipidomic analysis

Cell pellets were dissolved in 1 mL of methanol for every $10^6$ cells, vortexed, and homogenized. After homogenization, analyses were performed by hydrophilic interaction chromatography-tandem mass spectrometry (HILIC/MS/MS), lipidomics, and gas chromatography-tandem mass spectrometry (GC/MS/MS). Three lots of T cells, as well as transgenic CAR-T cells from each T cell, were used as samples, and the sampling was performed on days 1, 2, 3, and 4 after gene transfer.

In HILIC/MS/MS, 100 $\mu$L of the homogenate was centrifuged at 21,500 g for 5 minutes at 4°C. Then, 76 $\mu$L of the obtained supernatant was mixed with 4 $\mu$L of 400 mM ammonium formate. The mixture was vortexed and centrifuged at 21,500 g for 5 minutes at 4°C. The supernatant (2 $\mu$L) was injected into a liquid chromatography-tandem mass spectrometry (LC/MS/MS) system composed of a UHPLC Nexera liquid chromatography system (Shimadzu Corporation) and a 5500 QTRAP mass spectrometer (AB Sciex Pte. Ltd.). Chromatographic separation was performed on a ZIC-cHILIC column (2.1 $\times$ 100 mm, 3 $\mu$m, Merck Millipore). Selected reaction monitoring (SRM) was used to detect metabolites, and the abundance of each metabolite was evaluated as the peak area on the SRM chromatogram.

Lipidomics was performed by analyzing 2 $\mu$L of supernatant obtained by centrifuging the homogenate with an LC/MS/MS system composed of an Ultimate 3000 RSLC system (Thermo Fisher Scientific) and a Q Exactive HF-X mass spectrometer (Thermo Fisher Scientific). The separation by liquid chromatography was performed on a reversed-phase column CORTECS T3 (2.1 $\times$ 50 mm, 2.7 $\mu$m, 120 Å, Waters).

The GC/MS/MS analysis was performed under a nitrogen stream by drying 10 $\mu$L of supernatant obtained by centrifuging the homogenate, followed by derivatization by a two-step reaction of oximation and trimethylsilylation. Then, 1 $\mu$L of the reaction mixture was injected into an Agilent 7890B series gas chromatography system by using a GC Injector 80 autosampler (Agilent Technologies). The gas chromatographic separation was performed on a J&W Scientific DB-5MS-DG column (30 m $\times$ 0.25 mm i.d., df = 0.25 $\mu$m, Agilent Technologies). Each target molecule was detected by SRM, and the peak area was evaluated using MassHunter software (Agilent Technologies).

Enrichment and network analysis of lipids

The results of metabolomic and lipidomic analysis were analyzed using MetENP of an R package (doi: https://doi.org/10.1101/2020.11.20.391912).

Quantification of mitochondria with MitoTracker™

MitoTracker Deep Red (Thermo Fisher Scientific) was used for the measurement according to the manual.

Quantification of autofluorescence

Using flow cytometry, unstained cells were excited with laser light at wavelengths of 488 nm and 633 nm, and autofluorescence was measured in fluorescence channels with center wavelengths of 530 nm, 585 nm, and 660 nm.

Imaging by holographic microscope

The cells were imaged with a 3D Cell Explorer (Nanolive SA) according to the manual.

Total glycomics

After pretreatment of the cells, N-linked glycans were measured by cleaving the target glycans by trypsin/PNGaseF (FUJIFILM Wako Pure Chemical Corporation), capturing the glycans by glycoblotting, and labeling with aoWR(H) (Sumitomo Bakelite Co., Ltd.), followed by measurement by MALDI-TOF MS. Glycosphingolipid (GSL) glycans and free oligosaccharides (FOS) were measured by cleaving the target glycans by EGCaseI (New England Biolabs), capturing the glycans by glycoblotting, and labeling with aoWR(H), followed by measurement by MALDI-TOF MS. Glycosaminoglycans (GAG) were degraded to Δ2 sugar by Chondroitinase ABC (Sigma-Aldrich) or Heparinase I, II, III (Sigma-Aldrich), and the Δ2 sugar was captured on beads by glycoblotting, 2AB-labeled, and measured by UPLC. The O-linked glycans were measured by performing labeling according to a BEP method in which addition of 3-methyl-1-phenyl-5-pyrazolone by a β-elimination reaction in the presence of pyrazolone is performed simultaneously with the Michael addition to the glycan binding site of the deglycosylated peptide, followed by measurement by MALDI-TOF MS.

Example 1: Correlation between CAR gene transfer efficiency and SSC of CAR-T cells

CAR-T cells were produced, and the complexity of the cells as a final product after production was measured by flow cytometry. The results are shown in Fig. 1. The CAR gene transfer was confirmed to increase SSC (Figs. 1A-D). As a result, it was confirmed that SSC was correlated with CAR transfer efficiency (Fig. 1E, N=5, y=0.0429x+0.6086, $R^2$=0.0998). Additionally, whether prediction of the CAR transfer efficiency was possible from the mean, variance, median, mode, skewness, and kurtosis of the SSC distribution was evaluated. The results demonstrated that as the CAR transfer efficiency increased, the mean, variance, median, and mode increased while the skewness and kurtosis decreased (Fig. 1F). These results suggested that the use of all of these indexes enables prediction of CAR transfer efficiency.

Example 2: Prediction of gene transfer efficiency using SK-Hep-1 cells

Using the hepatocellular carcinoma cell line SK-HEP-1, it was confirmed whether the CAR gene transfer used in Example 1 would change the cell complexity, regardless of the type of cells for transfer (i.e., other than T cells). The results are shown in Fig. 2. As in Example 1, the CAR gene transfer was confirmed to increase SSC (Figs. 2A-D). As a result, it was confirmed that SSC was correlated with CAR transfer efficiency (Fig. 2E, N=4, y=0.0088x+0.8339, $R^2$=0.09912).

Example 3: Correlation between CAR gene transfer efficiency and SFL of CAR-T cells

In Examples 1 and 2, the cell complexity was measured using SSC; however, in order to confirm whether the cell complexity would also increase according to CAR gene transfer efficiency even when the cell complexity was based on the nucleic acid amount stained with a polymethine dye, the complexity of CAR-T cells was measured using XN-330. The results are shown in Fig. 3. The CAR gene transfer was confirmed to increase SFL (Figs. 3A-B). As a result, it was confirmed that SFL was correlated with CAR transfer efficiency (Fig. 3C, N=5, y=4.1959e$^{0.0166x}$, $R^2$=0.09942). Additionally, whether prediction of the CAR transfer efficiency was possible from the mean, variance, median, mode, skewness, and kurtosis of the SFL distribution was evaluated. The results demonstrated that as the CAR transfer efficiency increased, the mean, variance, median, and mode increased while the skewness and kurtosis decreased (Fig. 3D). These results suggested that the use of all of these indexes enables prediction of CAR transfer efficiency.

Example 4: Prediction of gene transfer efficiency from SSC distribution

The CAR transfer efficiency and SSC of CAR-T cells were measured by flow cytometry, a mixed lognormal distribution model consisting of two larger and smaller populations was fitted to the SSC distribution by maximum likelihood estimation, and the ratio of each population was calculated. The results are shown in Fig. 4. The percentage of the high-SSC cell population (67.5%) calculated using the mixed lognormal distribution model was very close to the CAR gene transfer efficiency (66.8%) measured by flow cytometry.

Example 5: Prediction of gene transfer efficiency using machine learning model

The SSC and FSC of CAR-T cells, as well as CAR-T cells co-expressing IL-7 and CCL19 (T cells transduced with the CAR gene, IL-7 gene, and CCL19 gene described above) were measured by flow cytometry, and the CAR gene transfer efficiency was predicted using a machine learning algorithm from the height, width, and area of the measured SSC and FSC (SSC-H, SSC-W, SSC-A, FSC-H, FSC-W, and FSC-A). The results are shown in Fig. 5. It was confirmed that the CAR gene transfer efficiency predicted using the machine learning algorithm from SSC-H, SSC-W, and SSC-A was correlated with the CAR gene transfer efficiency measured by flow cytometry in a conventional method (Fig. 5A, $R^2$=0.59). Furthermore, a more accurate prediction of the CAR gene transfer efficiency was possible with the machine learning algorithm using FSC-H, FSC-W, and FSC-A, in addition to SSC-H, SSC-W, and SSC-A (Fig. 5B, $R^2$=0.72).

Example 6: Structural comparison of cell membrane between CAR-T cells and untransduced T cells

The structural analysis of the cell membrane of CAR-T cells transduced with CAR gene and untransduced T cells as a control was performed by antibody staining (Figs. 6A-B) and electron microscopy (Figs. 6C-D). As a result, it was confirmed that the structure of the cell membrane was more complex in the CAR-T cells compared to the control (the arrows shown in Fig. 6). In addition, for the complexity of the cell membrane, image analysis was performed to quantify the area, diameter, circumference, and solidity (Fig. 6E). The results of the image analysis also demonstrated that the CAR-T cells had a longer circumference and a smaller solidity compared to those of the control, confirming that the structure of the cell membrane of the CAR-T cells became more uneven and more complex.

Example 7: Comparison of the cell membrane structural complexity between T cells and CAR-T cells

In order to study the complexity of the cell membrane, the morphology of the cell membrane was observed under transmission electron microscopy and scanning electron microscopy. After producing CAR-T cells or CAR-T cells expressing IL-7 and CCL19, only CAR-expressing cells were concentrated with MACS. Thereafter, imaging was performed with a transmission electron microscope (Fig. 7A) or a scanning electron microscope (Fig. 7B). The results demonstrated that the CAR-T cells or CAR-T cells expressing IL-7 and CCL19 had a more complex cell membrane structure, showing more protrusion structures than T cells.

Example 8: Changes in lipid components of cell membrane in T cells and CAR-T cells

In order to quantitatively observe the complexity of the cell membrane, metabolomic and lipidomic analysis was performed to quantify the membrane components. Three lots of T cells, as well as transgenic CAR-T cells from each T cell, were used as samples, and the sampling was performed on days 1, 2, 3, and 4. The results are shown in Figs. 8-10. The abbreviations for the lipid components in the figures are as follows: PA: phosphatidic acid, PC: phosphatidylcholine, LPC: lysophosphatidylcholine, PE: phosphatidylethanolamine, LPE: lysophosphatidylethanolamine, PG: phosphatidylglycerol, PI: phosphatidylinositol, PS: phosphatidylserine, SM: sphingomyelin, MG: monoacylglycerol, TG: triacylglycerol, Cer: ceramide, CL: cardiolipin, DHSM: dihydrosphingomyelin, FFA: free fatty acid, GM3: ganglioside M3, Hex-Cer: hexosylceramide, and Hex2-Cer: dihexosylceramide. As a more detailed classification, "a" represents an ester bond and "e" represents an ether bond between glycerol and a fatty acid, and the number of "a" or "e" indicates the number of fatty acid residues bound to the glycerol moiety. Additionally, the total carbon number of the binding fatty acid residues (x) and the number of double bonds (y) are expressed as x:y. For example, "PC (aa-34:1)" indicates that two fatty acids are ester bound to the glycerol moiety of phosphatidylcholine, that the total carbon number of fatty acid residues is 34, and that there is one double bond present therein.

Each target molecule was calculated as a value relative to the average of a QC sample obtained by mixing all samples. A t-test was performed on the values of the components on day 4 of expanded culture, and significantly different components were extracted (Fig. 8). A two-way analysis of variance was performed on the differences in the amounts of the components in the T cells and in the CAR-T cells produced from each T cell by the number of days of culture and the type of cells, and significantly different components were extracted (Fig. 9). Additionally, a principal component analysis was performed on the values of the components on day 4 of the expanded culture, and a scatter plot was plotted with the first principal component on the horizontal axis and the second principal component on the vertical axis (Fig. 10). This analysis revealed that the configuration of the membrane components was changed in T cells and CAR-T cells. It was also revealed that the component amount per cell increased in the CAR-T cells, indicating that the amount of the cell membrane per cell increased.

Example 9: Quantification of gene transfer efficiency using mitochondrial difference

Enrichment analysis (Fig. 11) and network analysis were performed using the results of the metabolomic and lipidomic analysis obtained in Example 8. In the figure, "TAG" represents triacylglycerol and "O-PC" represents ether phosphatidylcholine. The enrichment analysis clarified that the lipid subclasses of PG and PE in particular are upregulated in CAR-T cells. The network analysis clarified that pathways involved in the glycerophospholipid metabolism, glycosylphosphatidylinositol (GPI)-anchor biosynthesis, and glycerolipid metabolism were changed. These pathways have been reported to be involved in cell cycle initiation and T cell activation (Journal of Lipid Research, Volume 54, Issue 10, pp. 2665-2677 (2013), Biochemistry (Moscow) 81, 636-650 (2016)). Since PG and PE are known as lipids abundant in mitochondria (Journal of Japanese Biochemical Society, Vol. 83, No. 6, pp. 462-474, 2011, Biochimica et Biophysica Acta 1859 (2017) 1558-1572), it was assumed that the mitochondrial amount increased due to the gene transfer. Therefore, the mitochondrial capacity was quantified using a Mito-Tracker. The results confirmed that the gene transfer increased the mitochondrial capacity. These results clarified that the cells differed in complexity (Fig. 12). Mitochondria are known to emit autofluorescence when exposed to fluorescent wavelengths. Therefore, it was confirmed whether prediction of the CAR gene transfer efficiency was possible using autofluorescence. As a result, it was confirmed that the CAR gene transfer efficiency was correlated with autofluorescence (Fig. 13). These results clarified that the mitochondrial complexity, including autofluorescence, enables prediction of the gene transfer efficiency.

Example 10: Comparison of intracellular structural complexity in T cells and CAR-T cells

The intracellular structural complexity of CAR-T cells transduced with CAR gene and untransduced T cells as a control were compared under a holographic microscope. The results are shown in Fig. 14. The results confirmed more vacuoles and oil droplet-like structures in the CAR-T cells than in T cells. This indicated that the CAR-T cells became more complex.

Example 11: Changes in complexity in $\gamma\delta$ T cells transduced with mCherry gene

After the production of $\gamma\delta$ T cells transduced with mCherry gene, SSC was compared between mCherry-negative cells and mCherry-positive cells (Fig. 15A). The results demonstrated an increase in SSC in the mCherry-positive cells, indicating that the mCherry transfer increased the complexity of the $\gamma\delta$ T cells (Figs. 15B and 15C).

Example 12: Changes in complexity in $\gamma\delta$ T cells transduced with CAR gene

After the production of $\gamma\delta$ T cells transduced with CAR gene, SSC was compared between CAR-negative cells and CAR-positive cells (Fig. 16A). The results demonstrated an increase in SSC in the CAR-positive cells, indicating that the CAR gene transfer increased the complexity of the $\gamma\delta$ T cells (Fig. 16B).

Example 13: Changes in complexity in CAR-NK cells

After the production of CAR-NK cells transduced with CAR gene from NK92 cells, SSC was compared between CAR-negative cells and CAR-positive cells (Fig. 17A). The results demonstrated an increase in SSC in the CAR-positive cells, indicating that the CAR gene transfer increased the complexity of the NK92 cells (Fig. 17B).

Example 14: Total glycomics analysis with T cells and CAR-T cells

Glycans are present inside and outside cells. In order to study the complexity of cells, an analysis of a total of 171 types of glycans, including N-linked glycans, O-linked glycans, glycosphingolipid (GSL) glycans, glycosaminoglycans (GAG), and free oligosaccharides (FOS), was performed with CAR-T cells transduced with CAR gene and untransduced T cells as a control. As a result, the T cells and CAR-T cells showed different glycan profiles (Fig. 18A). In particular, N-linked glycans and O-linked glycans increased in T cells, while GSL, GAG, and FOS increased in the CAR-T cells (Fig. 18B). These results clarified that the cells differ in complexity. The following are glycans that showed different expression levels.
GLS-27; (Hex)3 (HexNAc)2 (NeuAc)1[a2,6], FOS-14; (Hex)5 (HexNAc)2, FOS-15; (Hex)6 (HexNAc)2, GLS-34; (Hex)4 (HexNAc)2 (NeuAc)1[a2,3], GLS-35; (Hex)3 (HexNAc)2 (Deoxyhexose)1 (NeuGc 26)1, FOS-05; (Hex)5 (HexNAc)1, FOS-04; (Hex)4 (HexNAc)1, GAG-03; ΔUA-GalNAc,4S, FOS-06; (Hex)6 (HexNAc)1, GAG-01;ΔUA-GalNAc, GLS-01; (Hex)2, FOS-13; (Hex)4 (HexNAc)2, N-40; (Hex)2 (HexNAc)3 (Deoxyhexose)1 + (Man)3 (GlcNAc)2, N-70; (Hex)3 (HexNAc)4 (Deoxyhexose)1 (NeuAc)1[a2,6] + (Man)3 (GlcNAc)2, N-54; (Hex)2 (HexNAc)3 (Deoxyhexose)1 (NeuAc)1 [a2,6]+ (Man)3 (GlcNAc)2, O-08; (Hex)1 (HexNAc)1 (NeuAc)2, N-11; (Hex)1 (HexNAc)1 + (Man)3 (GlcNAc)2, N-50; (Hex)2 (HexNAc)3 (NeuAc)1[a2,6] + (Man)3 (GlcNAc)2, N-68; (Hex)2 (HexNAc)3 (Deoxyhexose)1 (NeuAc)2[a2,6/a2,6] + (Man)3 (GlcNAc)2, N-71; (Hex)4 (HexNAc)4 (NeuAc)1[a2,6] + (Man)3 (GlcNAc)2, N-77; (Hex)4 (HexNAc)4 (Deoxyhexose)1 (NeuAc)1 [a2,6] + (Man)3 (GlcNAc)2, N-32; (Hex)2 (HexNAc)3 + (Man)3 (GlcNAc)2, N-02; (Man)2 (GlcNAc)2, N-33; (HexNAc)4 (Deoxyhexose)1 + (Man)3 (GlcNAc)2, N-31; (Hex)1 (HexNAc)3 (Deoxyhexose)1 + (Man)3 (GlcNAc)2, N72; (Hex)4 (HexNAc)5 (Deoxyhexose)1 + (Man)3 (GlcNAc)2, N-82; (Hex)3 (HexNAc)4 (Deoxyhexose)1 (NeuAc)2[a2,6/a2,6] + (Man)3 (GlcNAc)2, N-23; (Hex)1 (HexNAc)2 (Deoxyhexose)1 + (Man)3 (GlcNAc)2, N-04; (Man)3 (GlcNAc)2, N-06; (Hex)1 + (Man)3 (GlcNAc)2

The present application is based on Japanese Patent Application No. 2022-59600 filed on March 31, 2022, and Japanese Patent Application No. 2022-158218 filed on September 30, 2022, in Japan, the entire contents of which are incorporated herein.

Sequence Listing

**Claims**

1. A method for predicting the gene transfer efficiency of animal cells, comprising:

   (1) measuring the cell complexity of transgenic animal cells; and
   (2) predicting the gene transfer efficiency based on values measured in step (1).

2. The method according to claim 1, wherein the cell complexity is intracellular complexity.

3. The method according to claim 1, wherein in step (2), the distribution of cell complexities is used to predict the gene transfer efficiency of a cell population.

4. The method according to claim 1, wherein in step (2), a parameter of the distribution of cell complexities is used to predict the gene transfer efficiency of a cell population.

5. The method according to claim 4, wherein the parameter of the distribution of cell complexities is at least one member selected from the group consisting of mean, median, mode, variance, kurtosis, skewness, maximum value, minimum value, quartile, peak height, and half width.

6. The method according to claim 4, wherein the parameter of the distribution of cell complexities is at least one member selected from the group consisting of mean, median, mode, variance, kurtosis, and skewness.

7. The method according to claim 3, wherein in step (2), the distribution of complexities of transfected animal cells is determined from the distribution of cell complexities to thereby predict the gene transfer efficiency of the cell population.

8. The method according to claim 1, wherein the measurement of the cell complexity in step (1) is performed by measuring side scatter (SSC) or side fluorescence light (SFL) by a flow cytometer.

9. The method according to claim 1, wherein the measurement of the cell complexity in step (1) is performed by measuring SSC or SFL by a flow cytometer, and in step (2), SSC or SFL pulse height, width, and area are used to predict the gene transfer efficiency of the cell population.

10. The method according to claim 1, wherein in step (2), cell size values are further used to predict the gene transfer efficiency of the cell population.

11. The method according to claim 10, wherein forward scatter (FSC) values measured by a flow cytometer are used as the cell size values.

12. The method according to claim 11, wherein in step (2), FSC pulse height, width, and area are used to predict the gene transfer efficiency of the cell population.

13. The method according to claim 1, wherein the cell complexity is cell surface complexity.

14. The method according to claim 13, wherein the cell surface complexity is a circumference, solidity, unevenness, arithmetic average roughness, maximum height, ten-point average roughness, average spacing of unevenness, average spacing of local peaks, load length ratio, fractal dimension, aspect ratio, circularity, roundness, or compactness of the cells.

15. The method according to claim 1, wherein the measurement of the cell complexity in step (1) is performed by measuring intracellular organelles, cell surface glycans, cell membranes, cellular lipids, intracellular metabolites, cellular lipid droplets, or cell surface shapes.

16. The method according to claim 1, wherein the measurement of the cell complexity in step (1) is performed by measuring cell-derived autofluorescence.

17. The method according to claim 1, wherein the animal cells are immune cells.

18. The method according to claim 17, wherein the immune cells are T cells or NK cells.

19. The method according to claim 1, wherein the animal cells are epithelial cells.

20. The method according to claim 1, wherein a nucleic acid to be introduced is a nucleic acid encoding a chimeric antigen receptor.

21. The method according to claim 1, wherein the cell complexity difference between transfected cells and non-transfected cells is 5% or more for SSC or SFL values measured by a flow cytometer.

**22.** A method for producing a transgenic cell preparation, comprising:

(I) measuring the complexity of transgenic animal cells and measuring gene transfer efficiency based on measured values.

Fig. 1-1

A   Control

B   +CAR gene

C   Control

D   +CAR gene

Fig. 1-2

E

F

Fig. 2

Fig. 3-1

A  Control

B  +CAR gene

C

$$y = 4.1959e^{0.0166x}$$
$$R^2 = 0.9942$$

Fig. 3-2

D

EP 4 502 172 A1

Fig. 4

CAR positivity rate (flow cytometry): 66.8%
Predicted value: 67.5%

—— Measured value
········ Mixed lognormal distribution (predicted value)

Density

1.5e-05
1.0e-05
5.0e-06
0.0e+00

0
50000
100000
150000
200000
250000

SSC-A

Fig. 5

A

## Prediction from only SSC

R2=0.59

CAR positivity rate (flow cytometry)

B ## Prediction from both FSC and SSC

R2=0.72

CAR positivity rate (flow cytometry)

Fig. 6

Fig. 7A

Fig. 7B

| T-cell | CAR-T cell | 7x19 CAR-T cell |
|---|---|---|

EP 4 502 172 A1

Fig. 8B

Fig. 8C

EP 4 502 172 A1

Fig. 8D

Fig. 8F

EP 4 502 172 A1

Fig. 9A

Fig. 9B

Fig. 10

Fig. 11

Fig. 12

Fig. 13

Fig. 14

Fig. 15

Fig. 16

A

B

Fig. 17

A

B

Fig. 18

A

B

## INTERNATIONAL SEARCH REPORT

| International application No. |
| --- |
| **PCT/JP2023/013374** |

**A. CLASSIFICATION OF SUBJECT MATTER**

*C12Q 1/06*(2006.01)i; *C12N 5/0783*(2010.01)i; *C12N 5/10*(2006.01)i; *G01N 15/14*(2006.01)i; *G01N 33/483*(2006.01)i; *G01N 33/543*(2006.01)i; *C12N 15/09*(2006.01)n

FI: C12Q1/06 ZNA; C12N5/10; C12N5/0783; G01N33/483 C; G01N15/14 C; G01N33/543 597; C12N15/09 Z

According to International Patent Classification (IPC) or to both national classification and IPC

**B. FIELDS SEARCHED**

Minimum documentation searched (classification system followed by classification symbols)

C12Q1/06; C12N5/0783; C12N5/10; G01N15/14; G01N33/483; G01N33/543; C12N15/09

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | US 2019/0226994 A1 (CELLTOOL GMBH) 25 July 2019 (2019-07-25) | 1-7, 13-15, 17-20, 22 |
| | entirety in particular, claims, paragraphs [0062], [0070], [0076], [0083], [0086], examples | |
| Y | | 8-12, 21 |
| A | | 16 |
| Y | HIRAMATSU, K. et al. High-throughput label-free molecular fingerprinting flow cytometry. Science Advances. 2019, vol. 5, no. eaau0241, pp. 1-8 | 8-12, 21 |
| | abstract, p. 2, fig. 1 | |
| X | WANG, Y. et al. Regulation of gene transfection by cell size, shape and elongation on micropatterned surfaces. Journal of Materials Chemistry B. 2021, vol. 9, pp. 4329-4339 | 1-22 |
| | entirety in particular, abstract, p. 4332, p. 4334, fig. 2 | |
| A | WO 2022/045334 A1 (TAKEDA PHARMACEUTICAL COMPANY LIMITED.) 03 March 2022 (2022-03-03) | 1-22 |
| | claims, examples | |

☐ Further documents are listed in the continuation of Box C. ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **08 June 2023** | **20 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 502 172 A1**

**INTERNATIONAL SEARCH REPORT**

International application No.

**PCT/JP2023/013374**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex C/ST.25 text file" is replaced with the "form of ST.26."

Form PCT/ISA/210 (continuation of first sheet) (January 2015)

**INTERNATIONAL SEARCH REPORT**
Information on patent family members

International application No.

**PCT/JP2023/013374**

| Patent document cited in search report | | | Publication date (day/month/year) | Patent family member(s) | | | Publication date (day/month/year) |
|---|---|---|---|---|---|---|---|
| US | 2019/0226994 | A1 | 25 July 2019 | WO | 2018/007415 | A1 | |
| | | | | EP | 3479102 | A1 | |
| | | | | CN | 109690294 | A | |
| WO | 2022/045334 | A1 | 03 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022059600 A

- JP 2022158218 A

**Non-patent literature cited in the description**

- *Clinical Cytometry*, vol. 100 (2), 218-224
- *Journal of Lipid Research*, 2013, vol. 54 (10), 2665-2677
- *Biochemistry*, 2016, vol. 81, 636-650

- *Journal of Japanese Biochemical Society*, 2011, vol. 83 (6), 462-474
- *Biochimica et Biophysica Acta*, 2017, vol. 1859, 1558-1572